# EUROPEAN PATENT SPECIFICATION

(11) **EP 3 694 536 B1**
(45) Date of publication and mention of the grant of the patent: **27.08.2025**
(21) Application number: 18866399.1
(22) Date of filing: 12.10.2018
(51) Int. Cl.: A61K 38/16, A61K 38/17, A61K 49/00, C12N 15/62, G01N 33/50, G01N 33/68

(54) **A CELL-BASED SEEDING ASSAY FOR HUNTINGTIN AGGREGATION**
ZELLENBASIERTES IMPFASSAY ZUR HUNTINGTON-AGGREGATION
DOSAGE D'ENSEMENCEMENT BASÉ SUR DES CELLULES POUR L'AGRÉGATION DE LA HUNTINGTINE

(30) Priority: 12.10.2017 US 201762571443 P
(43) Date of publication of application: 19.08.2020
(73) Proprietor: The Regents of the University of California, Oakland, CA 94607 (US)
(72) Inventor: YANG, Xiangdong, W., Los Angeles CA 90095 (US); LEE, Chung-Ying, Torrance CA 90501 (US); WANG, Nan, Los Angeles CA 90095 (US); DAMOISEAUX, Robert, Los Angeles CA 90095 (US)
(74) Representative: Leonard, Thomas Charles
(86) International application number: PCT/US2018/055575
(87) International publication number: WO 2019/075302

(56) References cited:
- WO-A1-2015/161254
- WO-A2-2010/015592
- WO-A2-2014/145975
- WO-A2-2014/182972
- HOLLOSCHI ANDREAS ET AL: "X-ALD gene View project Membrane Alternatives View project", 1 September 2014 (2014-09-01), XP055821889, Retrieved from the Internet <URL:https://www.researchgate.net/publication/272161228_Analysis_of_huntingtin_aggregation_by_fluorescence_and_FRET_microscopy> [retrieved on 20210707]
- HERRERA F.: "Visualization of cell-to-cell transmission of mutant huntingtin oligomers", 11 February 2011 (2011-02-11), XP055821887, Retrieved from the Internet <URL:https://www.ncbi.nlm.nih.gov/pmc/articles/PMC3037516/> [retrieved on 20210707]
- GU ET AL.: "N17 Modifies mutant Huntingtin nuclear pathogenesis and severity of disease in HD BAC transgenic mice", NEURON, vol. 85, no. 4, 5 February 2015 (2015-02-05), pages 726 - 741, XP029198989, DOI: 10.1016/j.neuron.2015.01.008

## Description

### CROSS REFERENCE TO RELATED APPLICATIONS

This application claims priority to U.S. Provisional Application No. 62/571,443, filed October 12, 2017.

### STATEMENT REGARDING FEDERALLYSPONSORED RESEARCH OR

### DEVELOPMENT

This invention was made with government support under R01NS074312-03S1 awarded by the National Institutes of Health (NIH). The government has certain rights in the invention.

### BACKGROUND OF THE INVENTION

Huntington's disease (HD) is caused by CAG-repeat expansion mutation encoding an elongated polyglutamine (polyQ) repeat near the N-terminus of a variant or mutant Huntingtin (mHTT). The polyQ expansion increases tendency to form aggregates.

Several methods have been developed to detect HTT proteins in the biofluids. Direct detection, including MSD and Singulex, using antibody based method is limited by the specificity of the antibody. However, due to the size of mHTT (over 300 kDa) and extensive post-translational modifications plus conformation changes after oligomerization and aggregation, each antibody could only detect a subset of mHTT species. Currently, these methods only detect soluble forms, mostly monomers, of mHTT.

Nucleation/Seeding-induced aggregation methods include in vitro aggregation of purified HTT monomers and cell-based assays. In vitro aggregation assays are limited by a lack of sensitivity as they failed to detect any signal from biofluids. A cell-based seeding strategy was also limited as it showed spontaneous progressive aggregation at the base line and could not differentiate Aβ and HTT aggregates. It is also worth to note that there is not a high-throughput cell based assay available for detection of mHTT aggregation. Rather, a filter-trap method with Western blot-like detection is needed for the quantitation in current cell based assays providing a system that lacks sensitive and is labor-intensive.

There is thus a need in the art for high-throughput cell based assays for accurately detecting the presence of mHTT aggregates in biosamples for use in diagnosing Huntington's Disease. The present invention addresses this unmet need in the art.

### SUMMARY OF THE INVENTION

The invention is defined in the appended set of claims. The description may contain additional technical information, which although not part of the claimed invention, is provided to place the invention in a broader technical context and to illustrate possible related technical developments. Any reference in this specification to a method of treatment is to be interpreted as referring to the composition for use in the recited treatment

The present invention provides a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

In one embodiment, the mHTT lacks at least one function of the first 17 amino acid residues (N17) of exon 1 of HTT. In one embodiment, the mHTT lacks at least one of a signal for targeting the mHTT to the endoplasmic reticulum (ER) and a signal for exporting the mHTT from the nucleus.

In one embodiment, the mHTT comprises a fragment of exon 1 of HTT comprising a polyQ region having 46 consecutive glutamine amino acids and further lacking amino acid residues 2 through 16 of exon 1 of HTT.

In one embodiment, the nucleotide sequence encoding the mHTT is operably linked to a nucleotide sequence encoding a detectable marker. In one embodiment, the detectable marker is at least one marker selected from the group consisting of a fluorescent protein, an enzyme, and an epitope.

The present invention also provides a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

In one embodiment, the mHTT lacks at least one function of the first 17 amino acid residues (N17) of exon 1 of HTT. In one embodiment, the mHTT lacks at least one of a signal for targeting the mHTT to the endoplasmic reticulum (ER) and a signal for exporting the mHTT from the nucleus.

In one embodiment, the mHTT comprises a fragment of exon 1 of HTT comprising a polyQ region having 46 consecutive glutamine amino acids and further lacking amino acid residues 2 through 16 of exon 1 of HTT.

In one embodiment, the cell is a mammalian cell.

In one embodiment, the protein is a fusion protein comprising a detectable protein.

In one embodiment, the detectable protein is at least one protein selected from the group consisting of a fluorescent protein, an enzyme, and an epitope.

In one embodiment, the fluorescent protein is selected from the group consisting of a red fluorescent protein, a green fluorescent protein, a blue fluorescent protein, a yellow fluorescent protein, and a cyan fluorescent protein.

In one embodiment, the fluorescent protein is EGFP.

In one embodiment, the cell is an isolated cell.

The present invention also provides an isolated protein comprising a mutant huntingtin protein (mHTT) or fragment thereof, wherein the mHTT comprises a fragment of exon 1 of HTT comprising a polyQ region consisting of 46 consecutive glutamine amino acids.

In one embodiment, the mHTT lacks at least one function of the first 17 amino acid residues (N17) of exon 1 of HTT. In one embodiment, the mHTT lacks at least one of a signal for targeting the mHTT to the endoplasmic reticulum (ER) and a signal for exporting the mHTT from the nucleus. In one embodiment, the mHTT comprises a fragment of exon 1 of HTT comprising a polyQ region having 46 consecutive glutamine amino acids and further lacking amino acid residues 2 through 16 of exon 1 of HTT.

In one embodiment, the protein is a fusion protein comprising an amino acid sequence of mHTT fused to an amino acid sequence of a detectable marker. In one embodiment, the detectable marker at least one marker selected from the group consisting of a fluorescent protein, an enzyme, and an epitope.

The present invention also provides a method of identifying a compound that prevents or treats at least one of huntingtin aggregation, seeding and cytoxicity, the method comprising: culturing a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids, in the presence of a candidate agent under conditions that allow for aggregation of the mHTT, measuring aggregation of the mHTT in the presence of the candidate agent, and comparing mHTT aggregation measured in the presence of the candidate agent to mHTT aggregation measured in the absence of the candidate agent, wherein if mHTT aggregation measured is reduced in the presence of the candidate agent as compared to in the absence of the candidate agent, then the candidate agent is identified as a compound that prevents or treats mHTT aggregation.

In one embodiment, the conditions that allow for aggregation of the mHTT comprise contacting the cell with a sample from a subject having HD.

The present invention also provides a method of diagnosing a subject as having Huntington's Disease (HD) comprising contacting a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids with a sample from a subject, detecting aggregation of the mHTT in the presence of the sample, and diagnosing the subject as having HD or at risk of developing HD when mHTT aggregation is detected.

In one embodiment, the sample is selected from the group consisting of CSF and blood.

In one embodiment, the method further comprises treating or preventing HD, the method comprising administering to an individual having, or at risk of developing, HD a pharmaceutical composition comprising a therapeutically effective amount of a compound for the treatment or prevention of HD.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.
Figure 1, comprising Figure 1A through Figure 1C, depicts schematic diagrams demonstrating seed-induced aggregation of mHTT. Figure 1A depicts a schematic diagram demonstrating the generation of HTTexon1-Q51 seeds using sonicaton. Figure 1B depicts an exemplary diagram demonstrating that the use of seeds decreases the minimum time required do detect aggregation. Figure 1C depicts a diagram of the HTT exon1 46Q construct used in the cell based seeding assay.
Figure 2 depicts exemplary immunofluorescent images demonstrating HEK293 cells stably transfected with three modified mHTT-exon1-46Q-GFP constructs and their response to different HTTexon1-Q51 species.
Figure 3 depicts exemplary experimental results demonstrating that brain lysates from HD patients can seed HTT-GFP aggregation.
Figure 4 depicts exemplary experimental results demonstrating that the seeding effect is specific to HTT seeds.
Figure 5 depicts exemplary experimental results demonstrating that HTT seeding is specific to HD cerebrospinal fluid (CSF) samples.
Figure 6, comprising Figure 6A through Figure 6B, depicts experimental results demonstrating that the exemplary experimental results of HTT-GFP aggregation blocked by specific antibodies. Figure 6A depicts exemplary experimental results demonstrating that anti-polyP antibodies significantly reduced HTT-GFP aggregation induced by HTT seeds. Figure 6B depicts exemplary experimental results demonstrating that immunodepletion of seedable mHTT species from HD CSF significantly reduced its capability to induce HTT-GFP aggregation.

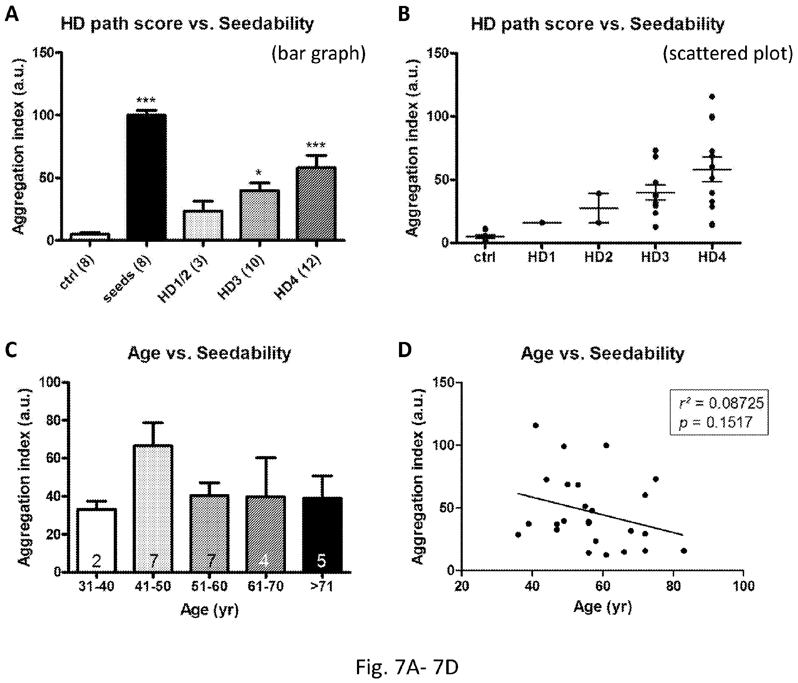

Figure 7, comprising Figure 7A through Figure 7D, depicts exemplary experimental results demonstrating that the postmortem CSF from HD subjects was able to induce HTT-GFP aggregation in the cell based assay. A total of 25 postmortem CSF samples from HD patients were used. Figure 7A depicts exemplary experimental results demonstrating the average seedability of CSF correlates to its HD pathology score. Figure 7B depicts exemplary experimental results demonstrating seedability of individual CSF samples vs. its HD pathology score. Figure 7C and 7D depict exemplary experimental results that the seedability of CSF is not correlated to the age of the individuals.

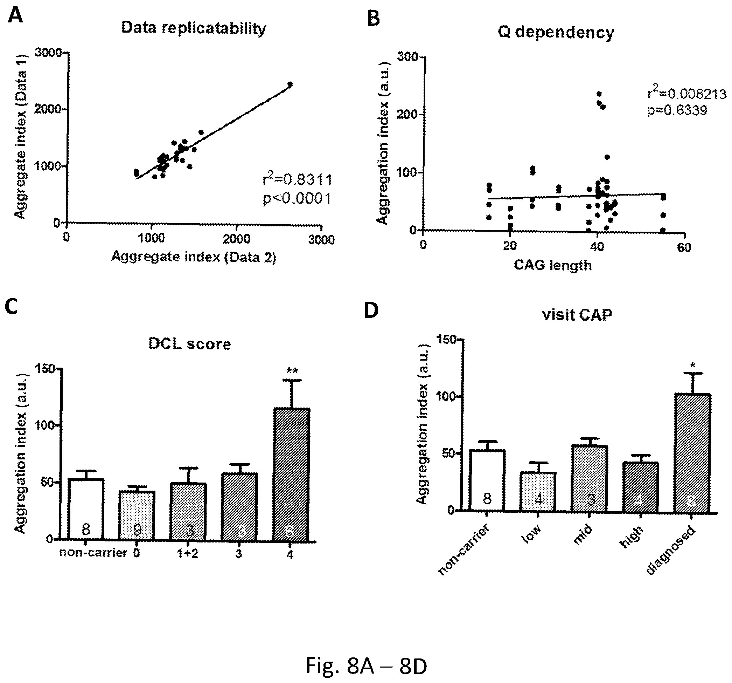

Figure 8, comprising Figure 8A through Figure 8D, depicts exemplary experimental results demonstrating that CSF seedability is significantly enhanced in samples from diagnosed or close-to-onset patients. A total of 33 CSF samples from HTT mutation carriers and non-carrier controls were evaluated. Figure 8A depicts exemplary experimental results demonstrating that the results from the seeding assay are highly replicable. Figure 8B depicts exemplary experimental results demonstrating that no Q length dependency was observed in the seeding assay. Figure 8C depicts exemplary experimental results demonstrating the aggregate formation in samples correlating with different diagnostic confidence level (DCL) scores. Figure 8D depicts exemplary experimental results demonstrating the aggregate formation in samples with different visit CAG-Age Products (CAPs).

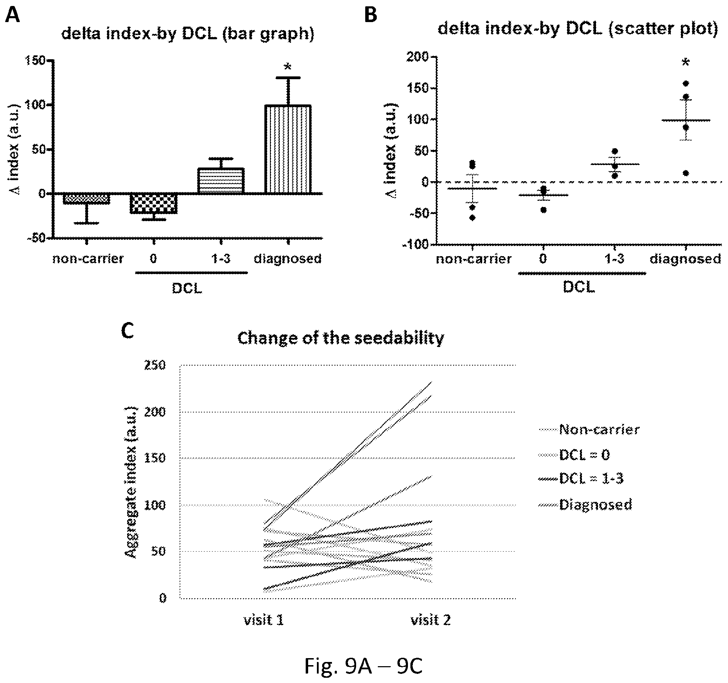

Figure 9, comprising Figure 9A through Figure 9C, depicts exemplary experimental results demonstrating that seedability increased with disease progress. A total of 33 CSF samples from HTT mutation carriers and non-carrier controls were evaluated. Figure 9A depicts exemplary experimental results demonstrating the average change in delta index of the sample groups with different DCL scores. Figure 9B depicts exemplary experimental results demonstrating the change in delta index from individual samples with different DCL scores. Figure 9C depicts exemplary experimental results demonstrating the change in aggregate formation between visits.
Figure 10 depicts exemplary experimental results demonstrating that overexpressing DNAJB6 significantly reduced HTT seeds- and HD CSF-induced HTT-GFP aggregation.
Figure 11 depicts exemplary experimental results demonstrating that the morphology of HTT-GFP aggregates is affected by the construct and conformation of the seeds administered.
Figure 12 depicts exemplary experimental results demonstrating that the sensitivity of the assay could be further enhanced by addition of the transfection reagent, FuGENE.

### DETAILED DESCRIPTION

The invention described herein provides new compositions and methods for rapidly evaluating huntingtin aggregation. The invention provides, accurate high-throughput methods for diagnosing the severity or progression of HD. The methods of the invention may be used in both clinical and research settings. The ability to diagnose the severity and progression of HD will enable physicians and other caregivers to best optimize HD therapeutic delivery and to evaluate other patient care needs. Likewise, the methods disclosed herein may be used for rapid or automated screening and evaluation of compounds that are being investigated as HD treatments.

The invention provides HTT variants that aggregate in the presence of HD bodily fluids such as CSF or blood, nucleic acid molecules that encode the HTT variants, HTT variant proteins, and cells expressing the HTT variants. The HTT variants may be expressed within cells, used in cell-free extracts, or used as synthetic peptide oligomers.

Specifically, the invention provides an isolated protein comprising a mutant huntingtin protein (mHTT) or fragment thereof, wherein the mHTT comprises a fragment of exon 1 of HTT comprising a polyQ region consisting of 46 consecutive glutamine amino acids. In one embodiment, the HTT variants comprise a fragment on the full length HTT protein. In one embodiment, the fragment comprises exon 1 of the full length HTT protein, or a fragment thereof. In one embodiment, the fragment of HTT comprises at least a PolyQ region of exon 1. In one embodiment, the fragment of HTT comprises at least a PolyQ region and a proline rich region of exon 1.

In one embodiment, the HTT variant or fragment thereof lacks at least one function of the first 17 amino acid residues of exon 1 of HTT (N17). In various embodiments, the HTT variant or fragment thereof lacks at least one of a signal for targeting HTT to the endoplasmic reticulum (ER) and a nuclear export signal. In one embodiment, the HTT variant or fragment thereof is not phosphorylated at at least one of S13 and S16 of exon 1. In one embodiment, the HTT variant or fragment thereof lacks at least one or both of S13 and S16 of exon 1. In one embodiment, the HTT variant or fragment thereof lacks a consensus or functional CRM1/exportin-dependent nuclear export signal.

In one embodiment, the HTT variant comprises a fragment of HTT lacking at least 1, 2, 3, 4, 5, 6, 7, 8, 9, 10, 11, 12, 13, 14, 15, 16, 17 or more than 17 amino acids of the N-terminus of exon 1. In one embodiment, the HTT variant comprises a fragment of exon 1 lacking at least 1, 2, 3, 4, 5, 6, 7, 8, 9, 10, 11, 12, 13, 14, 15, 16, 17 or more than 17 amino acids of the N-terminus of exon 1. In one embodiment, a fragment of HTT lacking at least 1, 2, 3, 4, 5, 6, 7, 8, 9, 10, 11, 12, 13, 14, 15, 16, 17 or more than 17 amino acids of the N-terminus of exon 1 is linked to an amino acid sequence or functional element at the N-terminus. In one embodiment, the fragment of HTT is linked to a methionine at the N-terminus.

In one embodiment, the HTT variants comprise greater than 20, 21, 22, 23, 24, 25, 26, 27, 28, 29, 30, 31, 32, 33, 34, 35, 36, 37, 38, 39, 40, 41, 42, 43, 44, or greater than 45 consecutive glutamine amino acids. In one embodiment, the HTT variants comprise less than 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, or less than 47 consecutive glutamine amino acids. In one embodiment, the HTT variants comprise about 46 consecutive glutamine amino acids.

In one embodiment, the HTT variants comprise a fragment of exon 1 of HTT comprising a polyQ region having 46 consecutive glutamine amino acids and further lacking amino acid residues 2 through 16 of exon 1 of HTT.

In one embodiment, the HTT variants are operably linked to one or more functional element or additional regulatory sequence. In various embodiments, an HTT variant of the invention may be operably linked to one or more of an N-terminal methionine, a purification tag, a reporter gene, a linker, a cleavage site, or any additional amino acid sequence.

In one embodiment, the invention contemplates isolated mHTT peptides. It also contemplates mHTT peptides expressed in cells and compositions, including cell lysates, comprising the mHTT peptides of the invention. The term peptide is meant to include a string of amino acids. The amino acids in the peptides of the invention may be naturally-occurring or non-naturally-occurring. The peptides of the invention may be synthesized chemically or biologically, and can include cysteine-rich peptides, circular peptides, stapled peptides, peptides that include D- or L-amino acids and mixtures thereof, peptidomimetics, peptide-nucleic acids (PNAs), and combinations thereof.

In one embodiment, the invention provides nucleic acid molecules encoding the HTT variants. In one embodiment, the nucleic acid molecule encodes a fragment on the full length HTT protein. In one embodiment, the fragment comprises exon 1 of the full length HTT protein, or a fragment thereof. In one embodiment, the fragment of exon 1 comprises at least a PolyQ region of exon 1 and a proline rich region of exon 1. In one embodiment, the nucleic acid molecule encodes a fragment of exon 1 lacking at least 1, 2, 3, 4, 5, 6, 7, 8, 9, 10, 11, 12, 13, 14, 15, 16, 17 or more than 17 amino acids of the N-terminus of exon 1. Specifically, the invention provides a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

In one embodiment, the nucleic acid molecule encodes greater than 20, 21, 22, 23, 24, 25, 26, 27, 28, 29, 30, 31, 32, 33, 34, 35, 36, 37, 38, 39, 40, 41, 42, 43, 44, or greater than 45 consecutive glutamine amino acids. In one embodiment, the nucleic acid molecule encodes less than 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, or less than 47 consecutive glutamine amino acids. In one embodiment, the nucleic acid molecule encodes about 46 consecutive glutamine amino acids. In one embodiment, the nucleic acid molecule encodes a fragment of exon 1 of HTT comprising a polyQ region having 46 consecutive glutamine amino acids and further lacking amino acid residues 2 through 16 of exon 1 of HTT.

In one embodiment the mHTT protein or fragment thereof comprises an amino acid sequence set forth in SEQ ID NO:2, SEQ ID NO:4 or SEQ ID NO:6. In one embodiment the mHTT protein or fragment thereof is encoded a nucleic acid sequence set forth in SEQ ID NO: 1, SEQ ID NO:3 or SEQ ID NO:5.

In some embodiments, the invention relates to recombinant cells that express a mHTT gene or a fragment thereof to produce a mHTT protein capable of forming aggregates.

Recombinant DNA technology is known in the art. In some embodiments, cells are transformed with expression vectors such as plasmids. In other embodiments, the vectors have one or more genetic signals, e.g., for transcriptional initiation, transcriptional termination, translational initiation and translational termination. In one embodiment, mHTT sequences may be cloned in a vector so that it is expressed when properly transformed into a suitable host organism. In some embodiments, the cells used in the methods disclosed herein utilize recombinant expression systems having elements as defined below:
In one embodiment, the encoding nucleotide sequence is operably linked to one or more functional element or regulatory sequence. In various embodiments, the encoding nucleotide sequence of the invention may be operably linked to one or more functional element or regulatory sequence including, but not limited to, a start codon, at least one stop codon, a sequence encoding a purification tag, a reporter gene, an antibiotic resistance gene, a restriction enzyme cleavage site, a promoter, and a Kozak sequence. The invention contemplates using any cells that are capable of expressing HTT and displaying aggregates in response to HD CSF.

In various embodiments, the invention relates to cells expressing a mHTT of the invention. Specifically, the invention provides a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids. Cell culture, cell line cultures, and tissue culture are known in the art. Cells can be isolated from tissues for ex vivo culture in several ways. Cells can be purified from blood. Mononuclear cells can be released from soft tissues by enzymatic digestion with enzymes such as collagenase, trypsin, or pronase, which break down the extracellular matrix. Alternatively, pieces of tissue can be placed in growth media, and the cells that grow out are available for culture.

Cells that are cultured directly from a subject are known as primary cells. With the exception of some derived from tumors, most primary cell cultures have limited lifespan. Primary cell cultures may be immortalized by techniques known in the art. An established or immortalized cell line has acquired the ability to proliferate indefinitely. Examples of know immortalization methods include isolation from a naturally occurring cancer, spontaneous or induced random mutagenesis, introduction of a viral gene or genome, artificial expression of key proteins, e.g. telomerase, and hybridoma technology. Additionally, unicellular organism as disclosed herein may be used for the assay methods described herein.

In some embodiments, the invention relates to methods of using mHTT variants or cells expressing mHTT variants of the invention for detection of HD. In one embodiment, the method comprises contacting a mHTT variant with a sample from a subject and detecting aggregation of the mHTT. Specifically, the invention provides a method of identifying a compound that prevents or treats at least one of huntingtin aggregation, seeding and cytoxicity, the method comprising: culturing a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids in the presence of a candidate agent under conditions that allow for aggregation of the mHTT, measuring aggregation of the mHTT in the presence of the candidate agent, and comparing mHTT aggregation measured in the presence of the candidate agent to mHTT aggregation measured in the absence of the candidate agent, wherein if mHTT aggregation measured is reduced in the presence of the candidate agent as compared to in the absence of the candidate agent, then the candidate agent is identified as a compound that prevents or treats mHTT aggregation.

In some embodiments, the method include using one or more controls in the HTT aggregation assay. Exemplary positive controls that may be used in the methods of the invention include, but are not limited to, CSF from known HD patients, HTT proteins that were isolated from HD CSF, HTT prepared from cells that have been modified or selected to produce the HTT proteins, and synthetic HTT proteins or HTT-like peptides that cause or trigger HTT aggregation via polyglutamine domains. Exemplary positive controls that may be used in the methods of the invention include, but are not limited to, non-HD CSF preparations, saline, solvents, diluents, and water, as is known in the art.

In some embodiments, the compositions disclosed herein are used in methods to identify therapeutic treatments for HD. Examples of therapeutic treatments can include small molecules, biologics, nucleic acids, cells, and viruses. Because the method is rapid, quantitative, and therapeutic, the progression or regression of HD can be monitored at time points chosen by a clinician or researcher. Likewise, the methods of the invention may be used to optimize or determine the efficacy of said therapeutic treatments. Treatment efficacy may be measured as part of a therapeutic regimen or during the process of treatment development, clinical trials, or other drug evaluations.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics which are normal or expected for one cell or tissue type, might be abnormal for a different cell or tissue type.

A "disease" is a state of health of an animal wherein the animal cannot maintain homeostasis, and wherein if the disease is not ameliorated then the animal's health continues to deteriorate.

In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

A disease or disorder is "alleviated" if the severity of a symptom of the disease or disorder, the frequency with which such a symptom is experienced by a patient, or both, is reduced.

An "effective amount" or "therapeutically effective amount" of a compound is that amount of compound which is sufficient to provide a beneficial effect to the subject to which the compound is administered. An "effective amount" of a delivery vehicle is that amount sufficient to effectively bind or deliver a compound.

"HTT" is the gene that encodes Huntingtin, the protein that, in certain forms, causes Huntington's disease. Non-diseased individuals have at the 5' end a repeating CAG sequence coding for the amino acid glutamine. This region is called a trinucleotide repeat. Normal persons have a CAG repeat count of between seven and 35 repeats. Higher repeat numbers are responsible for Huntington's disease and are referred to herein as mHTT. These aberrant polyglutamine domains cause aggregation of the Huntingtin protein. HTT and mHTT as used herein, refer to families of gene sequences that vary based upon the number of CAG repeats with HTT referring to sequences having a CAG repeat count of less than or equal to 35 repeats and mHTT referring to sequences having a CAG repeat count of greater than 35 repeats.

HTT refers to Huntingtin, a protein encoded by HTT and has an N-terminal polyglutamine domain that varies from individual to individual. HTT, as used herein, refers to a family of proteins that vary based upon the size of the polyglutamine domain. mHTT refers to mutant HTT proteins.

As used herein, an "instructional material" includes a publication, a recording, a diagram, or any other medium of expression which can be used to communicate the usefulness of a compound, composition, vector, or delivery system of the invention in the kit for effecting alleviation of the various diseases or disorders recited herein. Optionally, or alternately, the instructional material can describe one or more methods of alleviating the diseases or disorders in a cell or a tissue of a mammal. The instructional material of the kit of the invention can, for example, be affixed to a container which contains the identified compound, composition, vector, or delivery system of the invention or be shipped together with a container which contains the identified compound, composition, vector, or delivery system. Alternatively, the instructional material can be shipped separately from the container with the intention that the instructional material and the compound be used cooperatively by the recipient. The term "microarray" refers broadly to both "DNA microarrays" and "DNA chip(s)," and encompasses all art-recognized solid supports, and all art-recognized methods for affixing nucleic acid molecules thereto or for synthesis of nucleic acids thereon.

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.

A "polyadenylation signal" is a signal sequence which causes cleavage at a specific site at the 3' end of a eukaryotic mRNA molecule and involves a posttranscriptional incorporation of a sequence of about 100-200 adenine nucleotides (polyA tail) at the cleaved 3' end. The polyadenylation signal may comprise the sequence AATAAA about 10-30 nucleotides upstream of the cleavage site and a sequence located downstream. Various polyadenylation elements are known such as thymidine kinase (tk) polyA, SV40 late and early polyA or BGH polyA (described for example in U.S. Pat. No. 5,122,458).

A "promoter" refers to a polynucleotide sequence which allows and controls the transcription of the genes or sequences functionally connected to them. A promoter contains recognition sequences for binding RNA polymerase and the initiation site for transcription (transcription initiation site). A suitable functional promoter must be chosen. A variety of promoters from various sources are known to those of skill in the art. Promoters of the invention include constitutive, inducible and repressible promoters. The activity of inducible promoters is increased in response to cis or trans-acting factors or signals. Examples of inducible promoters are the jun, fos, metallothionein and heat shock promoters.

A "therapeutic" treatment is a treatment administered to a subject who exhibits signs of pathology, for the purpose of diminishing or eliminating those signs.

The phrase "therapeutically effective amount," as used herein, refers to an amount that is sufficient or effective to prevent or treat (delay or prevent the onset of, prevent the progression of, inhibit, decrease or reverse) a disease or condition associated with huntingtin aggregation, including alleviating symptoms of such diseases.

"Transcription-regulatory elements" generally refer to promoters upstream of the gene of interest to be expressed, transcription initiation and termination sites and a polyadenylation signal. Other transcription-regulatory elements include enhancers, locus control regions, and binding sites for cis or trans-acting factors.

The term "transcription initiation site" refers to a nucleic acid sequence that corresponds to the first nucleic acid residue that is transcribed into mRNA. The transcription initiation site may overlap with the promoter sequences.

The term "transcription termination site" refers to a nucleotide sequence that is normally at the 3' end of the nucleic acid sequence being transcribed and brings about the termination of transcription by RNA polymerase.

"Translation regulatory elements" comprise a translation initiation site (AUG), a stop codon and a polyA signal for each polypeptide to be expressed. In one embodiment, 5' or 3' untranslated regions of the nucleic acid sequence are added, removed, or changed in order to eliminate any potentially unsuitable additional translation initiation codons or other sequences which might affect expression at the transcription or expression level. In another embodiment, ribosomal consensus binding sites may be inserted immediately upstream of the start codon. Genes of interest encoding secreted proteins contain a signal precursor peptide which transports the synthesized polypeptide to and through the ER membrane. The signal sequence is often, but not always, located at the amino terminus of the secreted protein and may be cleaved by signal peptidases after the protein has passed through the ER membrane. The signal sequence may be native or heterologous to the gene of interest.

As used herein, "treating a disease or disorder" means reducing the frequency with which a symptom of the disease or disorder is experienced by a patient.

The terms "vector," "polynucleotide vector," "construct," and "polynucleotide construct" are used interchangeably herein. A polynucleotide vector of this invention may be in any of several forms, including, but not limited to, RNA, DNA, RNA encapsulated in a retroviral coat, DNA encapsulated in an adenovirus coat, DNA packaged in another viral or viral-like form (such as herpes simplex, and adeno-associated virus (AAV)), DNA encapsulated in liposomes, DNA complexed with polylysine, complexed with synthetic polycationic molecules, conjugated with transferring, complexed with compounds such as polyethylene glycol (PEG) to immunologically "mask" the molecule and/or increase half-life, or conjugated to a non-viral protein. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The invention provides, in part, a cell-based assay for detection of seedable HTT species, correlates to HD disease progression. In one embodiment, the invention provides constructs encoding mutant Huntingtin proteins and cells comprising the constructs for use in the cell-based assay of the invention. In one embodiment, the invention relates to methods of using the cell-based assay of the invention for diagnosing HD and for identifying compounds that could be effective in treating or preventing HD or an HD associated condition.

### Peptides

The present invention provides an isolated protein comprising a mutant huntingtin protein (mHTT) or fragment thereof, wherein the mHTT comprises a fragment of exon 1 of HTT comprising a polyQ region consisting of 46 consecutive glutamine amino acids.

The present invention comprises a peptide comprising a HTT variant or a fragment thereof. The peptide of the invention is sometimes referred to herein as mHTT. In one embodiment the mHTT comprises at least a polyQ and proline rich region of exon 1 of HTT.

The peptide of the present invention may be made using chemical methods. For example, peptides can be synthesized by solid phase techniques (Roberge J Y et al (1995) Science 269: 202-204), cleaved from the resin, and purified by preparative high performance liquid chromatography. Automated synthesis may be achieved, for example, using the ABI 431 A Peptide Synthesizer (Perkin Elmer) in accordance with the instructions provided by the manufacturer.

The peptide may alternatively be made by recombinant means or by cleavage from a longer polypeptide. The composition of a peptide may be confirmed by amino acid analysis or sequencing.

The variants of the polypeptides according to the present disclosure may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the polypeptide is an alternative splice variant of the polypeptide of the present invention, (iv) fragments of the polypeptides and/or (v) one in which the polypeptide is fused with another polypeptide, such as a leader or secretory sequence or a sequence which is employed for purification (for example, His-tag) or for detection (for example, Sv5 epitope tag). The fragments include polypeptides generated via proteolytic cleavage (including multi-site proteolysis) of an original sequence. Variants may be post-translationally, or chemically modified. Such variants are deemed to be within the scope of those skilled in the art from the teaching herein.

As known in the art the "similarity" between two polypeptides is determined by comparing the amino acid sequence and its conserved amino acid substitutes of one polypeptide to a sequence of a second polypeptide. Variants are defined to include polypeptide sequences different from the original sequence, preferably different from the original sequence in less than 40% of residues per segment of interest, more preferably different from the original sequence in less than 25% of residues per segment of interest, more preferably different by less than 10% of residues per segment of interest, most preferably different from the original protein sequence in just a few residues per segment of interest and at the same time sufficiently homologous to the original sequence to preserve the functionality of the original sequence and/or the ability to bind to ubiquitin or to a ubiquitylated protein. The present disclosure includes amino acid sequences that are at least 60%, 65%, 70%, 72%, 74%, 76%, 78%, 80%, 90%, or 95% similar or identical to the original amino acid sequence. The degree of identity between two polypeptides is determined using computer algorithms and methods that are widely known for the persons skilled in the art. The identity between two amino acid sequences is preferably determined by using the BLASTP algorithm [BLAST Manual, Altschul, S., et al., NCBI NLM NIH Bethesda, Md. 20894, Altschul, S., et al., J. Mol. Biol. 215: 403-410 (1990)].

The polypeptides of the invention can be post-translationally modified. For example, post-translational modifications that fall within the scope of the present invention include signal peptide cleavage, glycosylation, acetylation, isoprenylation, proteolysis, myristoylation, protein folding and proteolytic processing, etc. Some modifications or processing events require introduction of additional biological machinery. For example, processing events, such as signal peptide cleavage and core glycosylation, are examined by adding canine microsomal membranes or Xenopus egg extracts (U.S. Pat. No. 6,103,489) to a standard translation reaction.

The polypeptides of the invention may include unnatural amino acids formed by post-translational modification or by introducing unnatural amino acids during translation. A variety of approaches are available for introducing unnatural amino acids during protein translation. By way of example, special tRNAs, such as tRNAs which have suppressor properties, suppressor tRNAs, have been used in the process of sitedirected non-native amino acid replacement (SNAAR). In SNAAR, a unique codon is required on the mRNA and the suppressor tRNA, acting to target a non-native amino acid to a unique site during the protein synthesis (described in WO90/05785). However, the suppressor tRNA must not be recognizable by the aminoacyl tRNA synthetases present in the protein translation system. In certain cases, a non-native amino acid can be formed after the tRNA molecule is aminoacylated using chemical reactions which specifically modify the native amino acid and do not significantly alter the functional activity of the aminoacylated tRNA. These reactions are referred to as post-aminoacylation modifications. For example, the epsilon-amino group of the lysine linked to its cognate tRNA (tRNA_{LYS}), could be modified with an amine specific photoaffinity label.

Also contemplated within the scope of embodiments described herein are peptides that are branched or cyclic, with or without branching. Cyclic, branched and branched circular peptides result from post-translational natural processes and are also made by suitable synthetic methods. In some embodiments, any peptide product described herein comprises a peptide analog described above that is then covalently attached to an alkyl-glycoside surfactant moiety.

Also contemplated within the scope of embodiments presented herein are peptide chains that are substituted in a suitable position by the modification of the analogs claimed herein. For example, acylation is on a linker amino acid, for example, at the ε-position of Lysine, with fatty acids such as octanoic, decanoic, dodecanoic, tetradecanoic, hexadecanoic, octadecanoic, 3-phenylpropanoic acids and the like, or with saturated or unsaturated alkyl chains (Zhang, L. and Bulaj, G. (2012) Curr Med Chem 19: 1602-1618).

Also contemplated within the scope of embodiments presented herein are peptide chains that are comprised of natural and unnatural amino acids or analogs of natural amino acids. As used herein, peptide and/or protein "analogs" comprise nonnatural amino acids based on natural amino acids, such as tyrosine analogs, which includes para-substituted tyrosines, ortho-substituted tyrosines, and meta-substituted tyrosines, wherein the substituent on the tyrosine comprises an acetyl group, a benzoyl group, an amino group, a hydrazine, an hydroxyamine, a thiol group, a carboxy group, a methyl group, an isopropyl group, a C2-C20 straight chain or branched hydrocarbon, a saturated or unsaturated hydrocarbon, an O-methyl group, a polyether group, a halogen, a nitro group, or the like. Examples of Tyr analogs include 2,4-dimethyl-tyrosine (Dmt), 2,4-diethyl-tyrosine, O-4-allyl-tyrosine, 4-propyl-tyrosine, Ca-methyl-tyrosine and the like. Examples of lysine analogs include ornithine (Orn), homo-lysine, Ca-methyl-lysine (CMeLys), and the like. Examples of phenylalanine analogs include, but are not limited to, meta-substituted phenylalanines, wherein the substituent comprises a methoxy group, a C1-C20 alkyl group, for example a methyl group, an allyl group, an acetyl group, or the like. Specific examples include, but are not limited to, 2,4,6-trimethyl-L-phenylalanine (Tmp), O-methyl-tyrosine, 3-(2-naphthyl)alanine (Nal(2)), 3-(1-naphthyl)alanine (Nal(1)), 3-methyl-phenylalanine, 1,2,3,4-tetrahydroisoquinoline-3-carboxylic acid (Tic), fluorinated phenylalanines, isopropyl-phenylalanine, p-azido-phenylalanine, p-acyl-phenylalanine, p-benzoyl-phenylalanine, p-iodo-phenylalanine, p-bromophenylalanine, p-amino-phenylalanine, and isopropyl-phenylalanine, and the like.

Also contemplated within the scope of embodiments presented herein are peptide chains containing nonstandard or unnatural amino acids known to the art, for example, C-alpha-disubstituted amino acids such as Aib, Ca-diethylglycine (Deg), aminocyclopentane-1-carboxylic acid (Ac4c), aminocyclopentane-1-carboxylic acid (Ac5c), and the like. Such amino acids frequently lead to a restrained structure, often biased toward an alpha helical structure (Kaul, R. and Balaram, P. (1999) Bioorg Med Chem 7: 105-117). Additional examples of such unnatural amino acids useful in analog design are homo-arginine (Har), and the like. Substitution of reduced amide bonds in certain instances leads to improved protection from enzymatic destruction or alters receptor binding. By way of example, incorporation of a Tic-Phe dipeptide unit with a reduced amide bond between the residues (designated as Tic-F[CH₂-NH]-Phe) reduces enzymatic degradation.

Also contemplated within the scope of embodiments presented herein are modifications at the amino or carboxyl terminus may optionally be introduced into the present peptides or proteins (Nestor, J. J., Jr. (2009) Current Medicinal Chemistry 16: 4399-4418). For example, the present peptides or proteins can be truncated or acylated on the N-terminus (Gourlet, P., et al. (1998) Eur J Pharmacol 354: 105-1 1 1, Gozes, I. and Furman, S. (2003) Curr Pharm Des 9: 483-494).

### Fusion and Chimeric Polypeptides

A mHTT or fragment thereof of the invention may be conjugated with other molecules, such as proteins, to prepare fusion proteins. This may be accomplished, for example, by the synthesis of N-terminal or C-terminal fusion proteins.

Cyclic derivatives of the peptides or chimeric proteins of the invention are also part of the present invention. Cyclization may allow the peptide or chimeric protein to assume a more favorable conformation for association with other molecules. Cyclization may be achieved using techniques known in the art. For example, disulfide bonds may be formed between two appropriately spaced components having free sulfhydryl groups, or an amide bond may be formed between an amino group of one component and a carboxyl group of another component. Cyclization may also be achieved using an azobenzene-containing amino acid as described by Ulysse, L., et al., J. Am. Chem. Soc. 1995, 117, 8466-8467. The components that form the bonds may be side chains of amino acids, non-amino acid components or a combination of the two. In an embodiment of the invention, cyclic peptides may comprise a beta-turn in the right position. Beta-turns may be introduced into the peptides of the invention by adding the amino acids Pro-Gly at the right position.

It may be desirable to produce a cyclic peptide which is more flexible than the cyclic peptides containing peptide bond linkages as described above. A more flexible peptide may be prepared by introducing cysteines at the right and left position of the peptide and forming a disulphide bridge between the two cysteines. The two cysteines are arranged so as not to deform the beta-sheet and turn. The peptide is more flexible as a result of the length of the disulfide linkage and the smaller number of hydrogen bonds in the beta-sheet portion. The relative flexibility of a cyclic peptide can be determined by molecular dynamics simulations.

### (a) Tags

In a particular embodiment of the invention, the polypeptide of the invention further comprises the amino acid sequence of a tag. The tag includes but is not limited to: polyhistidine tags (His-tags) (for example H6 and H10, etc.), GST fusions, MBP fusions, streptavidine-tags, the BSP biotinylation target sequence of the bacterial enzyme BIRA and tag epitopes that are directed by antibodies (for example c-myc tags, FLAG-tags, among others). As will be observed by a person skilled in the art, the tag peptide can be used for purification, inspection, selection and/or visualization of the fusion protein of the invention. In a particular embodiment of the invention, the tag is a detection tag and/or a purification tag. It will be appreciated that the tag sequence will not interfere in the function of the protein of the invention.

In one embodiment, the invention provides HTT variants that further comprise a fluorescence tag or dye. Fluorescent tags are well-known in the art. A "fluorescent dye" or "fluorophore" or "fluorochrome" is a fluorescent chemical compound that can re-emit light upon light excitation. Fluorescent dyes typically, but not necessarily, contain several combined aromatic groups, or plane or cyclic molecules with several π bonds. In one embodiment, the HTT variants of the invention comprise a non-protein organic fluorophore. Non-protein organic fluorophores belong to following major chemical families: xanthene derivatives including fluorescein, rhodamine, Oregon green, eosin, and Texas red; cyanine derivatives including cyanine, indocarbocyanine, indocyanine green, oxacarbocyanine, thiacarbocyanine, and merocyanine; naphthalene derivatives including dansyl and prodan derivatives; coumarin derivatives; oxadiazole derivatives including pyridyloxazole, nitrobenzoxadiazole and benzoxadiazole; pyrene derivatives including cascade blue; oxazine derivatives including Nile red, Nile blue, cresyl violet, and oxazine 170; acridine derivatives incuding proflavin, acridine orange, and acridine yellow; arylmethine derivatives including auramine, crystal violet, and malachite green; and tetrapyrrole derivatives including porphin, phthalocyanine, and bilirubin. In one embodiment, the fluorescence tag is an enhanced green fluorescence protein (EGFP) and the mHTT or fragment thereof is fused to EGFP.

### (b) Leader and secretory sequences

Accordingly, the polypeptides of the invention can be fused to another polypeptide or tag, such as a leader or secretory sequence or a sequence which is employed for purification or for detection. In a particular embodiment, the polypeptide of the invention comprises the glutathione-S-transferase protein tag which provides the basis for rapid high-affinity purification of the polypeptide of the invention. Indeed, this GST-fusion protein can then be purified from cells via its high affinity for glutathione. Agarose beads can be coupled to glutathione, and such glutathione-agarose beads bind GST-proteins. Thus, in a particular embodiment of the invention, the polypeptide of the invention is bound to a solid support. In a preferred embodiment, if the polypeptide of the invention comprises a GST moiety, the polypeptide is coupled to a glutathione-modified support. In a particular case, the glutathione modified support is a glutathione-agarose bead. Additionally, a sequence encoding a protease cleavage site can be included between the affinity tag and the polypeptide sequence, thus permitting the removal of the binding tag after incubation with this specific enzyme and thus facilitating the purification of the corresponding protein of interest. Suitable protease cleavage sites for incorporation into the polypeptides of the invention include enterokinase, factor Xa, thrombin, TEV protease, PreScission protease, inteins and the like.

### (c) Targeting sequences

The invention also relates to novel proteins comprising a mHTT or fragment thereof of the invention fused to, or integrated into, a target protein, and/or a targeting domain capable of directing the chimeric protein to a desired cellular component or cell type or tissue. The chimeric proteins may also contain additional amino acid sequences or domains. The chimeric proteins are recombinant in the sense that the various components are from different sources, and as such are not found together in nature (i.e. are heterologous).

A target protein is a protein that is selected for degradation and for example may be a protein that is mutated or over expressed in a disease or condition. In another embodiment of the invention, a target protein is a protein that is abnormally degraded and for example may be a protein that is mutated or under-expressed in a disease or condition. The targeting domain can be a membrane spanning domain, a membrane binding domain, or a sequence directing the protein to associate with for example vesicles or with the nucleus. The targeting domain can target a mHTT or fragment thereof to a particular cell type or tissue. For example, the targeting domain can be a cell surface ligand or an antibody against cell surface antigens of a target tissue (e.g. neuron or tumor antigens). A targeting domain may target a mHTT or fragment thereof to a cellular component.

### (d) Peptide Mimetics

In other embodiments, the subject compositions are peptidomimetics of the mHTT or fragment thereof of the invention. Peptidomimetics are compounds based on, or derived from, peptides and proteins. The peptidomimetics of the present invention typically can be obtained by structural modification of a known mHTT sequence using unnatural amino acids, conformational restraints, isosteric replacement, and the like. The subject peptidomimetics constitute the continuum of structural space between peptides and non-peptide synthetic structures; mHTT peptidomimetics may be useful, therefore, in delineating pharmacophores and in helping to translate peptides into nonpeptide compounds with the activity of the parent peptides.

Moreover, as is apparent from the present disclosure, mimetopes of the subject mHTTs can be provided. Such peptidomimetics can have such attributes as being non-hydrolyzable (e.g., increased stability against proteases or other physiological conditions which degrade the corresponding peptide), increased specificity and/or potency, and increased cell permeability for intracellular localization of the peptidomimetic. For illustrative purposes, peptide analogs of the present invention can be generated using, for example, benzodiazepines (e.g., see Freidinger et al. in Peptides: Chemistry and Biology, G. R. Marshall ed., ESCOM Publisher: Leiden, Netherlands, 1988), substituted gama lactam rings (Garvey et al. in Peptides: Chemistry and Biology, G. R. Marshall ed., ESCOM Publisher: Leiden, Netherlands, 1988, p123), C-7 mimics (Huffman et al. in Peptides: Chemistry and Biologyy, G. R. Marshall ed., ESCOM Publisher: Leiden, Netherlands, 1988, p. 105), keto-methylene pseudopeptides (Ewenson et al. (1986) J Med Chem 29:295; and Ewenson et al. in Peptides: Structure and Function (Proceedings of the 9th American Peptide Symposium) Pierce Chemical Co. Rockland, Ill., 1985), β-turn dipeptide cores (Nagai et al. (1985) Tetrahedron Lett 26:647; and Sato et al. (1986) J Chem Soc Perkin Trans 1:1231), β-aminoalcohols (Gordon et al. (1985) Biochem Biophys Res Commun 126:419; and Dann et al. (1986) Biochem Biophys Res Commun 134:71), diaminoketones (Natarajan et al. (1984) Biochem Biophys Res Commun 124:141), and methyleneamino-modifed (Roark et al. in Peptides: Chemistry and Biology, G. R. Marshall ed., ESCOM Publisher: Leiden, Netherlands, 1988, p134). Also, see generally, Session III: Analytic and synthetic methods, in in Peptides: Chemistry and Biology, G. R. Marshall ed., ESCOM Publisher: Leiden, Netherlands, 1988)

In addition to a variety of side chain replacements which can be carried out to generate the mHTT peptidomimetics, the present disclosure specifically contemplates the use of conformationally restrained mimics of peptide secondary structure. Numerous surrogates have been developed for the amide bond of peptides. Frequently exploited surrogates for the amide bond include the following groups (i) transolefins, (ii) fluoroalkene, (iii) methyleneamino, (iv) phosphonamides, and (v) sulfonamides.

Moreover, other examples of mimetopes include, but are not limited to, protein-based compounds, carbohydrate-based compounds, lipid-based compounds, nucleic acid-based compounds, natural organic compounds, synthetically derived organic compounds, anti-idiotypic antibodies and/or catalytic antibodies, or fragments thereof. A mimetope can be obtained by, for example, screening libraries of natural and synthetic compounds for compounds capable of aggregating in the presence of a sample from a subject having HD. A mimetope can also be obtained, for example, from libraries of natural and synthetic compounds, in particular, chemical or combinatorial libraries (i.e., libraries of compounds that differ in sequence or size but that have the same building blocks). A mimetope can also be obtained by, for example, rational drug design. In a rational drug design procedure, the three-dimensional structure of a compound of the present invention can be analyzed by, for example, nuclear magnetic resonance (NMR) or x-ray crystallography. The three-dimensional structure can then be used to predict structures of potential mimetopes by, for example, computer modelling, the predicted mimetope structures can then be produced by, for example, chemical synthesis, recombinant DNA technology, or by isolating a mimetope from a natural source (e.g., plants, animals, bacteria and fungi).

A mHTT or fragment thereof of the invention may be synthesized by conventional techniques. For example, the peptides or chimeric proteins may be synthesized by chemical synthesis using solid phase peptide synthesis. These methods employ either solid or solution phase synthesis methods (see for example, J. M. Stewart, and J. D. Young, Solid Phase Peptide Synthesis, 2nd Ed., Pierce Chemical Co., Rockford Ill. (1984) and G. Barany and R. B. Merrifield, The Peptides: Analysis Synthesis, Biology editors E. Gross and J. Meienhofer Vol. 2 Academic Press, New York, 1980, pp. 3-254 for solid phase synthesis techniques; and M Bodansky, Principles of Peptide Synthesis, Springer-Verlag, Berlin 1984, and E. Gross and J. Meienhofer, Eds., The Peptides: Analysis, Synthesis, Biology, suprs, Vol 1, for classical solution synthesis.) By way of example, a mHTT or fragment thereof may be synthesized using 9-fluorenyl methoxycarbonyl (Fmoc) solid phase chemistry with direct incorporation of phosphothreonine as the N-fluorenylmethoxy-carbonyl-O-benzyl-L-phosphothreonine derivative.

N-terminal or C-terminal fusion proteins comprising a mHTT or fragment thereof of the invention conjugated with other molecules may be prepared by fusing, through recombinant techniques, the N-terminal or C-terminal of the mHTT or fragment thereof, and the sequence of a selected protein or selectable marker with a desired biological function. The resultant fusion proteins contain the mHTT or fragment thereof fused to the selected protein or marker protein as described herein. Examples of proteins which may be used to prepare fusion proteins include immunoglobulins, glutathione-S-transferase (GST), hemagglutinin (HA), and truncated myc.

Peptides of the invention may be developed using a biological expression system. The use of these systems allows the production of large libraries of random peptide sequences and the screening of these libraries for peptide sequences that bind to particular proteins. Libraries may be produced by cloning synthetic DNA that encodes random peptide sequences into appropriate expression vectors. (see Christian et al 1992, J. Mol. Biol. 227:711; Devlin et al, 1990 Science 249:404; Cwirla et al 1990, Proc. Natl. Acad, Sci. USA, 87:6378). Libraries may also be constructed by concurrent synthesis of overlapping peptides (see U.S. Pat. No. 4,708,871).

The mHTT or fragment thereof of the invention may be converted into pharmaceutical salts by reacting with inorganic acids such as hydrochloric acid, sulfuric acid, hydrobromic acid, phosphoric acid, etc., or organic acids such as formic acid, acetic acid, propionic acid, glycolic acid, lactic acid, pyruvic acid, oxalic acid, succinic acid, malic acid, tartaric acid, citric acid, benzoic acid, salicylic acid, benezenesulfonic acid, and toluenesulfonic acids.

### Nucleic Acids

The present invention provides a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

In one embodiment, the invention includes an isolated nucleic acid comprising a nucleotide sequence encoding a mHTT or fragment thereof.

The nucleotide sequences encoding a mHTT or fragment thereof can alternatively comprise sequence variations with respect to the original nucleotide sequences, for example, substitutions, insertions and/or deletions of one or more nucleotides, with the condition that the resulting polynucleotide encodes a polypeptide according to the invention. Therefore, the scope of the present invention includes nucleotide sequences that are substantially homologous to the nucleotide sequences recited herein and encodes a mHTT or fragment thereof.

In the sense used in this description, a nucleotide sequence is "substantially homologous" to any of the nucleotide sequences describe herein when its nucleotide sequence has a degree of identity with respect to the nucleotide sequence of at least 60%, advantageously of at least 70%, preferably of at least 85%, and more preferably of at least 95%. A nucleotide sequence that is substantially homologous to a nucleotide sequence encoding a mHTT or fragment thereof can typically be isolated from a producer organism of the polypeptide of the invention based on the information contained in the nucleotide sequence by means of introducing conservative or nonconservative substitutions, for example. Other examples of possible modifications include the insertion of one or more nucleotides in the sequence, the addition of one or more nucleotides in any of the ends of the sequence, or the deletion of one or more nucleotides in any end or inside the sequence. The degree of identity between two polynucleotides is determined using computer algorithms and methods that are widely known for the persons skilled in the art. The identity between two amino acid sequences is preferably determined by using the BLASTN algorithm [BLAST Manual, Altschul, S., et al., NCBI NLM NIH Bethesda, Md. 20894, Altschul, S., et al., J. Mol. Biol. 215: 403-410 (1990)].

In another aspect, the invention relates to a construct, comprising a nucleotide sequence encoding a mHTT or fragment thereof, or derivative thereof. In a particular embodiment, the construct is operatively bound to transcription, and optionally translation, control elements. The construct can incorporate an operatively bound regulatory sequence of the expression of the nucleotide sequence of the invention, thus forming an expression cassette.

A mHTT or fragment thereof may be prepared using recombinant DNA methods. Accordingly, nucleic acid molecules which encode a mHTT or fragment thereof may be incorporated in a known manner into an appropriate expression vector which ensures good expression of the mHTT or fragment thereof.

Therefore, in another aspect, the invention relates to a vector, comprising the nucleotide sequence of the invention or the construct of the invention. The choice of the vector will depend on the host cell in which it is to be subsequently introduced. In a particular embodiment, the vector of the invention is an expression vector. Suitable host cells include a wide variety of prokaryotic and eukaryotic host cells. In specific embodiments, the expression vector is selected from the group consisting of a viral vector, a bacterial vector and a mammalian cell vector. Prokaryote- and/or eukaryotevector based systems can be employed for use with the present invention to produce polynucleotides, or their cognate polypeptides. Many such systems are commercially and widely available.

Further, the expression vector may be provided to a cell in the form of a viral vector. Viral vector technology is well known in the art and is described, for example, in Sambrook et al. (2001), and in Ausubel et al. (1997), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers. (See, e.g., WO 01/96584; WO 01/29058; and U.S. Pat. No. 6,326,193.

Vectors suitable for the insertion of the polynucleotides are vectors derived from expression vectors in prokaryotes such as pUC18, pUC19, Bluescript and the derivatives thereof, mp18, mp19, pBR322, pMB9, ColE1, pCR1, RP4, phages and "shuttle" vectors such as pSA3 and pAT28, expression vectors in yeasts such as vectors of the type of 2 micron plasmids, integration plasmids, YEP vectors, centromere plasmids and the like, expression vectors in insect cells such as vectors of the pAC series and of the pVL, expression vectors in plants such as pIBI, pEarleyGate, pAVA, pCAMBIA, pGSA, pGWB, pMDC, pMY, pORE series and the like, and expression vectors in eukaryotic cells based on viral vectors (adenoviruses, viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAX1, pZeoSV2, pCI, pSVL and PKSV-10, pBPV-1, pML2d and pTDT1.

By way of illustration, the vector in which the nucleic acid sequence is introduced can be a plasmid which is or is not integrated in the genome of a host cell when it is introduced in the cell. Illustrative, non-limiting examples of vectors in which the nucleotide sequence of the invention or the gene construct of the invention can be inserted include a tet-on inducible vector for expression in eukaryote cells.

The vector may be obtained by conventional methods known by persons skilled in the art (Sambrook et al., "Molecular cloning, a Laboratory Manual", 2nd ed., Cold Spring Harbor Laboratory Press, N.Y., 1989 Vol 1-3]. In a particular embodiment, the vector is a vector useful for transforming animal cells.

The recombinant expression vectors may also contain nucleic acid molecules which encode a portion which provides increased expression of the recombinant mHTT or fragment thereof; increased solubility of the recombinant mHTT or fragment thereof; and/or aid in the purification of the recombinant mHTT or fragment thereof by acting as a ligand in affinity purification. For example, a proteolytic cleavage site may be inserted in the recombinant peptide to allow separation of the recombinant mHTT or fragment thereof from the fusion portion after purification of the fusion protein. Examples of fusion expression vectors include pGEX (Amrad Corp., Melbourne, Australia), pMAL (New England Biolabs, Beverly, Mass.) and pRIT5 (Pharmacia, Piscataway, N.J.) which fuse glutathione S-transferase (GST), maltose E binding protein, or protein A, respectively, to the recombinant protein.

Additional promoter elements, i.e., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either co-operatively or independently to activate transcription.

A promoter may be one naturally associated with a gene or polynucleotide sequence, as may be obtained by isolating the 5' non-coding sequences located upstream of the coding segment and/or exon. Such a promoter can be referred to as "endogenous." Similarly, an enhancer may be one naturally associated with a polynucleotide sequence, located either downstream or upstream of that sequence. Alternatively, certain advantages will be gained by positioning the coding polynucleotide segment under the control of a recombinant or heterologous promoter, which refers to a promoter that is not normally associated with a polynucleotide sequence in its natural environment. A recombinant or heterologous enhancer refers also to an enhancer not normally associated with a polynucleotide sequence in its natural environment. Such promoters or enhancers may include promoters or enhancers of other genes, and promoters or enhancers isolated from any other prokaryotic, viral, or eukaryotic cell, and promoters or enhancers not "naturally occurring," *i.e.,* containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR^{™}, in connection with the compositions disclosed herein (U.S. Patent 4,683,202, U.S. Patent 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the cell type, organelle, and organism chosen for expression. Those of skill in the art of molecular biology generally know how to use promoters, enhancers, and cell type combinations for protein expression, for example, see Sambrook et al. (2001). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

A promoter sequence exemplified in the experimental examples presented herein is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, Moloney virus promoter, the avian leukemia virus promoter, Epstein-Barr virus immediate early promoter, Rous sarcoma virus promoter, as well as human gene promoters such as, but not limited to, the actin promoter, the myosin promoter, the hemoglobin promoter, and the muscle creatine promoter. Further, the invention should not be limited to the use of constitutive promoters. Inducible promoters are also contemplated as part of the invention. The use of an inducible promoter in the invention provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter. Further, the invention includes the use of a tissue specific promoter, which promoter is active only in a desired tissue. Tissue specific promoters are well known in the art and include, but are not limited to, the HER-2 promoter and the PSA associated promoter sequences.

In a particular embodiment, the expression of the nucleic acid is externally controlled. In a more particular embodiment, the expression is externally controlled using the doxycycline Tet-On system.

The recombinant expression vectors may also contain a selectable marker gene which facilitates the selection of transformed or transfected host cells. Suitable selectable marker genes are genes encoding proteins such as G418 and hygromycin which confer resistance to certain drugs, β-galactosidase, chloramphenicol acetyltransferase, firefly luciferase, or an immunoglobulin or portion thereof such as the Fc portion of an immunoglobulin preferably IgG. The selectable markers may be introduced on a separate vector from the nucleic acid of interest.

Reporter genes are used for identifying potentially transfected cells and for evaluating the functionality of regulatory sequences. Reporter genes that encode for easily assayable proteins are well known in the art. In general, a reporter gene is a gene that is not present in or expressed by the recipient organism or tissue and that encodes a protein whose expression is manifested by some easily detectable property, e.g., enzymatic activity. Expression of the reporter gene is assayed at a suitable time after the DNA has been introduced into the recipient cells.

Suitable reporter genes may include genes encoding luciferase, betagalactosidase, chloramphenicol acetyl transferase, secreted alkaline phosphatase, or the green fluorescent protein gene (see, e.g., Ui-Tei et al., 2000 FEBS Lett. 479:79-82). Suitable expression systems are well known and may be prepared using well known techniques or obtained commercially. Internal deletion constructs may be generated using unique internal restriction sites or by partial digestion of non-unique restriction sites. Constructs may then be transfected into cells that display high levels of siRNA polynucleotide and/or polypeptide expression. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Recombinant expression vectors may be introduced into host cells to produce a recombinant cell. The cells can be prokaryotic or eukaryotic. The vector of the invention can be used to transform eukaryotic cells such as yeast cells, Saccharomyces cerevisiae, or mammal cells for example epithelial kidney 293 cells or U2OS cells, or prokaryotic cells such as bacteria, Escherichia coli or Bacillus subtilis, for example. Nucleic acid can be introduced into a cell using conventional techniques such as calcium phosphate or calcium chloride co-precipitation, DEAE-dextran-mediated transfection, lipofectin, electroporation or microinjection. Suitable methods for transforming and transfecting host cells may be found in Sambrook et al. (Molecular Cloning: A Laboratory Manual, 2nd Edition, Cold Spring Harbor Laboratory press (1989)), and other laboratory textbooks.

For example, a mHTT or fragment thereof of the invention may be expressed in bacterial cells such as E. coli, insect cells (using baculovirus), yeast cells or mammalian cells. Other suitable host cells can be found in Goeddel, Gene Expression Technology: Methods in Enzymology 185, Academic Press, San Diego, Calif. (1991).

### Cells

The present invention provides a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

A nucleic acid for expression of a mHTT or fragment thereof can be transfected into a mammalian cell using nucleic acid vectors that include, but are not limited to, plasmids, linear nucleic acid molecules, artificial chromosomes, and viral vectors.

In some embodiments, a cell a non-human mammal cell (e.g., mouse, rat, or guinea pig). In one embodiment, the cell is a human cell.

Once the vector or nucleic acid molecule containing the construct(s) has been prepared for expression, the DNA construct(s) can be introduced into an appropriate host cell by any of a variety of suitable means, i.e., transformation, transfection, conjugation, protoplast fusion, electroporation, particle gun technology, calcium phosphate-precipitation, direct microinjection, and the like. Where the vector is a viral vector, the vector can be delivered to the cell by direct infection. Preferably the vector is stably integrated into the host genome. Methods of producing a cell line containing a nucleic acid (e.g., a vector) are well known in the art (see, for example, Sambrook et al. in Molecular Cloning: A Laboratory Manual. Cold Spring Harbor Laboratory Press, NY, Vol. 1, 2, 3 (1989)). Briefly, a cell transfected with a nucleic acid can be selected for using a host of antibiotics including, for example, G418, neomycin, or hygromycin B. Generally, the transfected nucleic acid contains a suitable antibiotic resistance gene or is co-transfected with a vector containing a suitable antibiotic resistance gene. Thus, only cells and their progeny which contain a nucleic acid encoding the antibiotic resistance gene will survive when grown in antibiotic.

### Methods

The present invention relates, in part, to the use of the cell-based assay system of the invention for the identification of subjects having or at risk of developing HD or HD related conditions. Accordingly, the present invention features methods for identifying subjects having or at risk of developing HD or HD related conditions, including those subjects who are asymptomatic or only exhibit non-specific indicators of HD by detection of mHTT aggregation in a cell-based assay of the invention. The methods are also useful for monitoring subjects undergoing treatments and therapies for HD or HD related conditions, and for selecting or modifying therapies and treatments that would be efficacious in subjects having or at risk of developing HD or HD related conditions, wherein selection and use of such treatments and therapies slow the progression of HD or HD related conditions, or prevent their onset.

The present invention provides a method of identifying a compound that prevents or treats at least one of huntingtin aggregation, seeding and cytoxicity, the method comprising: culturing a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids in the presence of a candidate agent under conditions that allow for aggregation of the mHTT, measuring aggregation of the mHTT in the presence of the candidate agent, and comparing mHTT aggregation measured in the presence of the candidate agent to mHTT aggregation measured in the absence of the candidate agent, wherein if mHTT aggregation measured is reduced in the presence of the candidate agent as compared to in the absence of the candidate agent, then the candidate agent is identified as a compound that prevents or treats mHTT aggregation.

The method of identifying a subject as having or at risk of developing HD or HD related conditions can include measuring mHTT aggregation induced by contacting a cell of the invention with a sample from the subject, and comparing the measured values to reference or index values. Such a comparison can be undertaken with mathematical algorithms or formula. Subjects identified as having or at risk of developing HD or HD related conditions can optionally be selected to receive treatment regimens, such as administration of prophylactic or therapeutic compounds or implementation of exercise regimens or dietary supplements to prevent, treat or delay the onset of HD or HD related conditions.

Identifying a subject before they develop HD or HD related conditions enables the selection and initiation of various therapeutic interventions or treatment regimens in order to delay, reduce or prevent that subject's conversion to a disease state. Monitoring the levels of mHTT aggregation from multiple samples from the same subject also allows for the course of treatment of HD or HD related conditions to be monitored. For example, a sample can be provided from a subject undergoing treatment regimens or therapeutic interventions, e.g., drug treatments, for HD or HD related conditions. Such treatment regimens or therapeutic interventions can include exercise regimens, dietary modification, dietary supplementation, administration of pharmaceuticals, and treatment with therapeutics or prophylactics used in subjects diagnosed or identified with HD or HD related conditions. Samples can be obtained from the subject at various time points before, during, or after treatment.

The cell-based assay of the present invention can be used to generate a mHTT aggregation profile of subjects: (i) who do not have and are not expected to develop HD or HD related conditions and/or (ii) who have or expected to develop HD or HD related conditions. The mHTT aggregation profile of a subject can be compared to a predetermined or reference mHTT aggregation profile to diagnose or identify subjects at risk for developing HD or HD related conditions, to monitor the progression of disease, as well as the rate of progression of disease, and to monitor the effectiveness of HD or HD related condition treatments. Data concerning the mHTT aggregation of the present invention can also be combined or correlated with other data or test results, such as, without limitation, measurements of clinical parameters or other algorithms for HD or HD related conditions. Other data includes age, ethnicity, HD path scores, DCL score, and CAP. The other data can also comprise subject information such as medical history and any relevant family history.

The present invention also provides methods for identifying agents for treating HD or HD related condition treatments that are appropriate or otherwise customized for a specific subject. In this regard, a test sample from a subject, exposed to a therapeutic agent or a drug, can be taken and the level of mHTT aggregation that is induced from contact of a cell of the invention with the test sample can be determined. The level of mHTT aggregation that is induced can be compared to a sample derived from the subject before and after treatment, or can be compared to samples derived from one or more subjects who have shown improvements in risk factors as a result of such treatment or exposure.

In one embodiment, the invention is a method of diagnosing HD. Specifically the present invention provides a method of diagnosing a subject as having Huntington's Disease (HD) comprising contacting a cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids with a sample from a subject, detecting aggregation of the mHTT in the presence of the sample, and diagnosing the subject as having HD or at risk of developing HD when mHTT aggregation is detected. In various embodiments, methods are disclosed herein that may be of use to determine whether a subject has a specific stage of HD, for instance HD1/2, HD3 or HD4. In some embodiments, these methods may utilize a biological sample (such as CSF, urine, saliva, blood, serum, amniotic fluid, or tears), for the detection of the level of mHTT aggregation that is induced from contact of a cell of the invention with the test sample.

In one embodiment, the method comprises contacting a cell of the invention expressing mHTT with a biological sample of the subject. In one embodiment, the biological sample is CSF. In various embodiments, the level of mHTT aggregation in the cell contacted with the biological sample of the subject is compared with the level of mHTT aggregation in a comparator. Non-limiting examples of comparators include, but are not limited to, a negative control, a positive control, an expected normal background value of the subject, a historical normal background value of the subject, an expected normal background value of a population that the subject is a member of, or a historical normal background value of a population that the subject is a member of.

In another embodiment, the disclosure is a method of monitoring the progression of HD in a subject by assessing the level of mHTT aggregation that is induced from contact of a cell of the invention with a biological sample of the subject.

In various embodiments, the subject is a human subject, and may be of any race, sex and age.

Information obtained from the methods of the invention described herein can be used alone, or in combination with other information (e.g., disease status, disease history, vital signs, blood chemistry, etc.) from the subject or from the biological sample obtained from the subject.

In various embodiments, the level of mHTT aggregation is determined to be increased when the level of mHTT aggregation in a cell of the invention is increased by at least 2%, at least 3%, at least 4%, at least 5%, at least 6%, at least 7%, at least 8%, at least 9%,at least 10%, by at least 20%, by at least 30%, by at least 40%, by at least 50%, by at least 60%, by at least 70%, by at least 80%, by at least 90%, or by at least 100%, when contacted with a biological sample from a subject, compared to a comparator control.

### Detection of mHTT aggregation

The mHTT aggregates can be detected by, for example, any fixation and antibody-based staining methods described herein or known in the art. Alternatively, the protein or proteins can be detectable proteins (e.g., a green fluorescent protein or a protein conjugated to a fluorescent protein) in which case the proteins can be directly visualized in the cell. These methods can include, for example, fluorescence based detection methods, including immunofluorescence. Fluorescence based detection methods encompass known techniques in the art that are used for light microscopy with a fluorescence microscope. In one embodiment, the technique uses an antibody to detect mHTT aggregates. In another embodiment, immunofluorescence microscopy assays are used to count cells that have aggregates. Immunofluorescence microscopy is a widely used example of examining immunostained samples and is a specific example of immunohistochemistry that makes use of fluorophores to visualize the location of the antibodies.

In another embodiment, gel electrophoresis is used to identify HTT aggregates. Gel electrophoresis can identify HTT aggregates and may be detected by western blot or other well-known visualization or quantification techniques. Other embodiments for quantification include, filter trap and ELISA assays. In preferred embodiments, quantification may make use of trapping the HTT proteins with molecules that specifically bind it, such as anti-polyglutamine antibodies (see below). Other assays contemplated by the invention include XTT cell rescue assays and Flow cytometry.

In one embodiment, the methods disclosed herein may require one or more antibodies, derivatives, or antibody-like molecules. In one embodiment, antibodies directed to epitopes on the HTT molecule may be used. In one embodiment, a monoclonal antibody is used. In one embodiment, an immunoglobulin protein that specifically binds an epitope on HTT is used. In one embodiment, the mAb1574 monoclonal antibody is used.

The term antibody is meant to include monoclonal antibodies, polyclonal antibodies, antibodies, antibody fragments (e.g., Fc domains), Fab fragments, single chain antibodies, bi- or multi-specific antibodies, Llama antibodies, nano-bodies, diabodies, Fv, Fab, F(ab')2, Fab', scFv, scFv-Fc, and the like. Also included in the term are antibody-fusion proteins, such as Ig chimeras.

The terms "antibody" and "immunoglobulin" are used interchangeably in the broadest sense and include monoclonal antibodies (e.g., full length or intact monoclonal antibodies), polyclonal antibodies, monovalent antibodies, multivalent antibodies, multispecific antibodies (e.g., bispecific antibodies so long as they exhibit the desired biological activity) and may also include certain antibody fragments (as described in greater detail herein). An antibody can be chimeric, human, humanized and/or affinity matured.

The terms "full length antibody," "intact antibody" and "whole antibody" are used herein interchangeably to refer to an antibody in its substantially intact form, not antibody fragments as defined below. The terms particularly refer to an antibody with heavy chains that contain the Fc region. "Antibody fragments" comprise a portion of an intact antibody, preferably comprising the antigen binding region thereof. Examples of antibody fragments include Fab, Fab', F(ab')2, and Fv fragments; diabodies; linear antibodies; single-chain antibody molecules; and multispecific antibodies formed from antibody fragments. The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies.

In certain embodiments, such a monoclonal antibody typically includes an antibody comprising a polypeptide sequence that binds a target, wherein the targetbinding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

### CSF Sample

It has been surprisingly found that CSF from HD subjects is effective in inducing aggregation in the cells of the invention. Therefore, in one embodiment, the method relates to contacting a cell of the invention with a CSF sample from a subject and detecting mHTT aggregation induced in the cell. In one embodiment, a CSF sample from a subject is processed prior to use in the assay of the invention. In one embodiment, the CSF can be diluted at about one of the following ratios: 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20. In other embodiments, the dilution may fall within about one of the following ranges 1:20-1:30, 1:30-1:40, 1:40-1:50, 1:50-1:60, 1:60-1:70, 1:70-1:80, 1:80-1:90, 1:90-1:100, 1:100-1:200, 1:200-1:300, 1:300-1:400, 1:400-1:500, 1:500-1:600, 1:600-1:700, 1:700-1:800, 1:800-1:900, or 1:900-1:1000. In yet other embodiments, CSF may be used at a 10n-fold dilution wherein n is 3, 4, 5, 6, 7, 8, 9 or 10.

In some embodiments, it may be required to collect CSF. Methods for collecting CSF are well known in the art. One embodiment contemplates lumbar puncture with fluid collection. Other embodiments contemplate alternative methods of CSF collection that may be necessary, e.g., if the subject has a back deformity or an infection. Thus another embodiment contemplates cisternal puncture. This method uses a needle placed below the occipital bone, usually done with fluoroscopy. In another embodiment, ventricular puncture is used. This technique may be used with subjects having possible brain herniation. A hole is drilled in the skull, and a needle is inserted directly into one of the brain's ventricles. In yet another embodiment, CSF may also be collected from a tube that's already placed in the fluid, such as a shunt or a ventricular drain.

### Screening Methods

The invention features methods of screening for and identifying a "candidate agent" (e.g., a compound or a drug) that prevents or reduces mHTT aggregation. Thus, a "candidate agent," as referred to herein, is any substance with a potential to reduce, interfere with or curtail (i.e., prevent or suppress) mHTT aggregation. It should be understood that the agents identified by the screening methods herein could be useful in preventing or treating HD in a subject (e.g., a human patient).

Various types of candidate agents can be screened by the methods described herein, including, but not limited to, nucleic acids, polypeptides, small molecule compounds, large molecule compounds, peptidomimetics or any other compounds described herein (e.g., see "Compounds" below). In some instances, the candidate agents are genetic agents that reduce, interfere with, or curtail mHTT aggregation. For example, a library of siRNAs or antisense oligonucleotides can be screened such that the level or amount of mHTT aggregation could be determined in the presence of each. In these examples, a reduced level of mHTT aggregation in the absence of a gene (through mutational inactivation or silencing) indicates that the gene contributes to mHTT aggregation. Accordingly, siRNAs or antisense oligonucleotides that target that gene, for example, can be useful in treating or preventing HD.

Screening methods to identify an agent capable of preventing or reducing mHTT aggregation can involve the steps of: (i) culturing a cell expressing mHTT in the presence of a candidate agent and under conditions that allow for aggregation of the mHTT; (ii) measuring mHTT aggregation in the presence of the candidate agent; and (iii) comparing mHTT aggregation measured in the presence of the candidate agent to mHTT aggregation in the absence of the candidate agent, where if mHTT aggregation is reduced in the presence of the candidate agent as compared to in the absence of the candidate agent, then the candidate agent is identified as a compound that prevents or reduces mHTT aggregation.

It should be understood that the screening methods described herein can be used as cell-based screens to identify compounds useful in treating HD. For example, the screening methods can comprise contacting a cell having mHTT aggregates with an agent and measuring mHTT aggregates in the presence and absence of the agent, wherein a decrease in mHTT aggregation in the presence of the agent is an indicator that the agent is a compounds useful for treating HD.

The screening assays can involve a mammalian cell containing a nucleic acid encoding a mHTT. Although the cell can be any mammalian cell, in one embodiment the cell is a human cell.

Cells containing a nucleic acid encoding a mHTT can be grown in tissue culture plates, ideally, multi-well assay plates such as 96 well culture plates. Cells can be cultured in the absence (Uninduced) or presence (Induced) of an inducing agent (e.g., CSF from a subject diagnosed as having HD) to induce aggregation of mHTT. Uninduced and induced cells can also be treated with a candidate compound (e.g., one dose of a candidate agent, e.g., a compound). Induced or uninduced cells cultured in the absence of a compound can optionally be treated with a like amount or concentration of the medium in which the candidate agent was delivered (e.g., DMSO). The assay can include cells or sets of cells as follows: (i) Induced cells treated with a candidate compound; (ii) Induced cells not treated with a candidate compound; (iii) Uninduced cells treated with a candidate compound; and (iv) Uninduced cells not treated with a candidate compound. To determine if a candidate compound prevents or reduces mHTT aggregation, the amount of mHTT aggregation present in cell set (i) can be compared to the amount of mHTT aggregation in cell set (ii). If more mHTT aggregation is present in cell set (ii) than is present in cell set (i), this is an indication that the agent prevents or reduces mHTT aggregation. Cell sets (iii) and (iv) can be used as controls to, for example, normalize the amount of mHTT aggregation in cells due to the culture conditions irrespective of inducing agent or compound respectively. For example, the amount of mHTT aggregation present in cell set (iii) can indicate if a candidate agent itself induces mHTT aggregation.

The cells can be treated with two or more concentrations of a compound, where, for example, a concentration-dependence or EC50 is to be determined (see below). Suitable concentrations of a candidate compound for the assay include, for example, about 0.01 µM to 1 mM of the agent (e.g., about 0.01 µM to 0.1 µM, about 0.1 µM to 1 µM, about 1 µM to 10 µM, about 10 to 1 mM, or about 100 µM to 1 mM).

It is understood that some optimization can be required to determine a suitable amount of inducing agent or compound for the method. Such optimization can be based on, for example, the type of cell used, the specific compound, the amount of mHTT aggregation induction, or the time required before or after induction of mHTT aggregation.

Methods of assessing the efficacy of an agent to prevent or reduce mHTT aggregation can be quantitative, semi-quantitative, or qualitative. Thus, for example, the activity of an agent can be determined as a discrete value. An example of a quantitative determination of an agent's is a 50% Effective Concentration, or EC50 value, which is the molar concentration of an agent (e.g., a compound) that gives one-half the maximal response of that agent. Alternatively, the efficacy of an agent can be assessed using a variety of semi-quantitative/qualitative systems known in the art. Thus, the efficacy of an agent to prevent or reduces mHTT aggregation can be expressed as, for example, (a) one or more of "excellent", "good", "satisfactory", "unsatisfactory", and/or "poor"; (b) one or more of "very high", "high", "average", "low", and/or "very low"; or (c) one or more of "+++++", "++++", "+++", "++", "+", ".+-.", and/or "-".

Methods for determining the efficacy of agents in preventing or reducing mHTT aggregation (e.g., a compound such as any of those described herein) are also included. Cells are generally plated on solid support matrix (e.g., a plastic tissue culture plate, or a multi-well (96 or 386-well) tissue culture plate) and grown in appropriate medium. Cells are then contacted with serial dilutions of a candidate agent generally ranging, for example, from 10 µM to 0.1 µM concentration. Often, a control compound (e.g., a known inhibitor of known concentration) is also added to a set of cells as an internal standard. Often, a set of cells are grown in the presence of a carrier, buffer, or solvent, in which the compound is delivered. Cells are grown in the presence or absence of test compounds for varying times, for example, from 1 to three days (1 day, 2 days, 3 days, 4 days, 1 week, 2 weeks), followed by a test for the number of cells remaining on the plate or the viability of the cells remaining on the plate. Methods of detecting (e.g., determining or measuring) the extent of mHTT aggregation in the presence or absence of an agent are myriad and include those discussed elsewhere herein as well as methods well known to those of ordinary skill in the art.

Screening assays can be performed in any format that allows for rapid preparation, processing, and analysis of multiple reactions. This can be, for example, in multi-well assay plates (e.g., 96 wells or 386 wells). Stock solutions for various agents can be made manually or robotically, and all subsequent pipetting, diluting, mixing, distribution, washing, incubating, sample readout, data collection and analysis can be done robotically using commercially available analysis software, robotics, and detection instrumentation capable of detecting the signal generated from the assay. Examples of such detectors include, but are not limited to, flow cytometers and fluorimeters.

### Compounds

Compounds to be screened or identified using any of the methods described herein can include various chemical classes, though typically small organic molecules having a molecular weight in the range of 50 to 2,500 daltons. These compounds can comprise functional groups necessary for structural interaction with proteins (e.g., hydrogen bonding), and typically include at least an amine, carbonyl, hydroxyl, or carboxyl group, and preferably at least two of the functional chemical groups. These compounds often comprise cyclical carbon or heterocyclic structures and/or aromatic or polyaromatic structures (e.g., purine core) substituted with one or more of the above functional groups.

In alternative embodiments, compounds can also include biomolecules including, but not limited to, peptides, polypeptides, peptidomimetics (e.g., peptoids), amino acids, amino acid analogs, saccharides, fatty acids, steroids, purines, pyrimidines, derivatives or structural analogues thereof, polynucleotides, nucleic acid aptamers, and polynucleotide analogs.

Compounds can be identified from a number of potential sources, including: chemical libraries, natural product libraries, and combinatorial libraries comprised of random peptides, oligonucleotides, or organic molecules. Chemical libraries consist of diverse chemical structures, some of which are analogs of known compounds or analogs or compounds that have been identified as "hits" or "leads" in other drug discovery screens, while others are derived from natural products, and still others arise from non-directed synthetic organic chemistry. Natural product libraries re collections of microorganisms, animals, plants, or marine organisms which are used to create mixtures for screening by: (1) fermentation and extraction of broths from soil, plant or marine microorganisms, or (2) extraction of plants or marine organisms. Natural product libraries include polypeptides, non-ribosomal peptides, and variants (non-naturally occurring) thereof. For a review, see Science 282:63-68 (1998). Combinatorial libraries are composed or large numbers of peptides, oligonucleotides, or organic compounds as a mixture. These libraries are relatively easy to prepare by traditional automated synthesis methods, PCR, cloning, or proprietary synthetic methods. Of particular interest are nonpeptide combinatorial libraries. Still other libraries of interest include peptide, protein, peptidomimetic, multiparallel synthetic collection, recombinatorial, and polypeptide libraries. For a review of combinatorial chemistry and libraries created therefrom, see Myers, Curr. Opin. Biotechnol. 8:701-707 (1997). Identification of test compounds through the use of the various libraries herein permits subsequent modification of the test compound "hit" or "lead" to optimize the capacity of the "hit" or "lead" to prevent or reduce mHTT aggregation.

The compounds identified above can be synthesized by any chemical or biological method. The compounds identified above can also be pure, or may be in a heterologous composition (e.g., a pharmaceutical composition), and can be prepared in an assay-, physiologic-, or pharmaceutically-acceptable diluent or carrier (see Pharmaceutical Compositions and Methods of Treatment below).

### Pharmaceutical Compositions

An agent found to prevent or reduce mHTT aggregation can be formulated as a pharmaceutical composition, e.g., for administration to a subject diagnosed with having or at risk of developing HD. Typically, a pharmaceutical composition includes a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. The composition can include a pharmaceutically acceptable salt, e.g., an acid addition salt or a base addition salt (see e.g., Berge et al., J. Pharm. Sci. 66:1-19, 1977).

The agent can be formulated according to standard methods. Pharmaceutical formulation is a well-established art, and is further described, e.g., in Gennaro (ed.), Remington: The Science and Practice of Pharmacy, 20th ed., Lippincott, Williams & Wilkins (2000) (ISBN: 0683306472); Ansel et al., Pharmaceutical Dosage Forms and Drug Delivery Systems, 7th Ed., Lippincott Williams & Wilkins Publishers (1999) (ISBN: 0683305727); and Kibbe (ed.), Handbook of Pharmaceutical Excipients American Pharmaceutical Association, 3rd ed. (2000) (ISBN: 091733096X).

In one embodiment, an agent that prevents or reduces mHTT aggregation can be formulated with excipient materials, such as sodium chloride, sodium dibasic phosphate heptahydrate, sodium monobasic phosphate, and a stabilizer. It can be provided, for example, in a buffered solution at a suitable concentration and can be stored at 2-8°C.

The pharmaceutical compositions may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form can depend on the intended mode of administration and therapeutic application. Typically compositions for the agents described herein are in the form of injectable or infusible solutions.

Such compositions can be administered by a parenteral mode (e.g., intravenous, subcutaneous, intraperitoneal, or intramuscular injection). The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, intracerebral, intracranial, intracarotid and intrasternal injection and infusion. The composition can be formulated as a solution, microemulsion, dispersion, liposome, or other ordered structure suitable for stable storage at high concentration. Sterile injectable solutions can be prepared by incorporating an agent described herein in the required amount in an appropriate solvent with one or a combination of ingredients enumerated above, as required, followed by filtered sterilization. Generally, dispersions are prepared by incorporating an agent described herein into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of an agent described herein plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

In certain embodiments, the agent can be prepared with a carrier that will protect the compound against rapid release, such as a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known. See, e.g., Sustained and Controlled Release Drug Delivery Systems, J. R. Robinson, ed., Marcel Dekker, Inc., New York, 1978.

An agent identified as one that prevents or reduces mHTT aggregation can be modified, e.g., with a moiety that improves its stabilization and/or retention in circulation, e.g., in blood, serum, or other tissues, e.g., by at least 1.5, 2, 5, 10, or 50 fold.

For example, the agent can be associated with a polymer, e.g., a substantially non-antigenic polymer, such as a polyalkylene oxide or a polyethylene oxide. Suitable polymers will vary substantially by weight. Polymers having molecular number average weights ranging from about 200 to about 35,000 Daltons (or about 1,000 to about 15,000, and 2,000 to about 12,500) can be used. For example, an agent can be conjugated to a water soluble polymer, e.g., a hydrophilic polyvinyl polymer, e.g., polyvinylalcohol or polyvinylpyrrolidone. A non-limiting list of such polymers include polyalkylene oxide homopolymers such as polyethylene glycol (PEG) or polypropylene glycols, polyoxyethylenated polyols, copolymers thereof and block copolymers thereof, provided that the water solubility of the block copolymers is maintained. Additional useful polymers include polyoxyalkylenes such as polyoxyethylene, polyoxypropylene, and block copolymers of polyoxyethylene and polyoxypropylene (Pluronics); polymethacrylates; carbomers; and branched or unbranched polysaccharides. When the agent (e.g., a compound) is used in combination with a second agent (e.g., any additional therapies for synucleinopathies such as acetylcholinesterase inhibitors), the two agents can be formulated separately or together. For example, the respective pharmaceutical compositions can be mixed, e.g., just prior to administration, and administered together or can be administered separately, e.g., at the same or different times.

### Administration

An agent that prevents or reduces mHTT aggregation can be administered to a subject, e.g., a human subject, by a variety of methods. For many applications, the route of administration is one of: intravenous injection or infusion (IV), subcutaneous injection (SC), intraperitoneally (IP), or intramuscular injection. In some cases, administration may be directly into the CNS, e.g., intrathecal, intracerebroventricular (ICV), intracerebral or intracranial. The agent can be administered as a fixed dose, or in a mg/kg dose. In other instances, administration can be oral (e.g., inhalation), transdermal (topical), transmucosal, or rectal.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

The powders and tablets contain from 1% to 95% (w/w) of the active compound. **In** certain embodiments, the active compound ranges from 5% to 70% (w/w). Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

Where the agent is a polypeptide or otherwise particularly antigenic, the dose can also be chosen to reduce or avoid production of antibodies against the agent. The route and/or mode of administration of the agent can also be tailored for the individual case.

Dosage regimens are adjusted to provide the desired response, e.g., a therapeutic response or a combinatorial therapeutic effect. The dosage regimen will, for example, prevent or reduce mHTT aggregation in one or more affected cells in a mammal having HD. Generally, a dose of an agent (e.g., a compound) is optionally formulated separately or together with an appropriate dose of a second therapeutic agent can be used to provide a subject with the agent. Suitable dosages and/or dose ranges for the agent include an amount sufficient to prevent or reduce mHTT aggregation in a subject.

A dose of an agent required to prevents or reduces mHTT aggregation can depend on a variety of factors including, for example, the age, sex, and weight of a subject to be treated. Other factors affecting the dose administered to the subject include, e.g., the stage or severity of the HD. For example, a patient with advanced HD may require administration of a different dosage of an agent that prevents or reduces mHTT aggregation than a patient with early-stage HD. Other factors can include, e.g., other disorders concurrently or previously affecting the patient, the general health of the patient, the genetic disposition of the patient, diet, time of administration, rate of excretion, drug combination, and any other additional therapeutics that are administered to the patient. It should also be understood that a specific dosage and treatment regimen for any particular patient will depend upon the judgment of the treating physician. The amount of active ingredients will also depend upon the particular described compound and the presence or absence and the nature of the additional therapeutic agents in the composition.

Dosage unit form or "fixed dose" as used herein refers to physically discrete units suited as unitary dosages for the subjects to be treated; each unit contains a predetermined quantity of active compound calculated to produce the desired therapeutic effect (e.g., prevents or reduces mHTT aggregation) in association with the required pharmaceutical carrier and optionally in association with the other agent. Suitable administration frequencies are described elsewhere herein.

A pharmaceutical composition can include a therapeutically effective amount of an agent found to prevent or reduce mHTT aggregation described herein. Such effective amounts can be determined based on the effect of the administered agent, or the combinatorial effect of an agent and secondary agent if more than one agent is used. A therapeutically effective amount of an agent can also vary according to factors such as the disease state, age, sex, and weight of the individual, and the ability of the compound to elicit a desired response in the individual, e.g., amelioration of at least one disorder parameter, e.g., amelioration of at least one symptom of HD, e.g., chorea. A therapeutically effective amount is also one in which any toxic or detrimental effects of the composition is outweighed by the therapeutically beneficial effects.

### Treatments

Any reference in this specification to a method of treatment is to be interpreted as referring to the composition for use in the recited treatment. In one embodiment, the methods of the disclosure include providing a treatment or treatment regimen to a subject identified as having or at risk of developing HD or HD related conditions. Treatments that can be administered include, but are not limited to Tetrabenazine, antipsychotic drugs such as haloperidol and chlorpromazine, risperidone, quetiapine, amantadine, levetiracetam, clonazepam, antidepressants, olanzapine, valproate, carbamazepine, and amotrigine. Other treatments can include speech therapy, physical therapy, occupational therapy, or coping and support services.

In certain embodiments, treatment comprises administering a diseasemodulating drug to a subject. The drug can be a therapeutic or prophylactic used in subjects diagnosed or identified with a disease or at risk of having the disease. In certain embodiments, modifying therapy refers to altering the duration, frequency or intensity of therapy, for example, altering dosage levels.

In various embodiments, effecting a therapy comprises causing a subject to or communicating to a subject the need to make a change in lifestyle, for example, increasing exercise, changing diet, reducing or eliminating smoking and so on.

Measurement of biomarker levels allow for the course of treatment of a disease to be monitored. The effectiveness of a treatment regimen for a disease can be monitored by detecting one or more biomarkers in an effective amount from samples obtained from a subject over time and comparing the amount of biomarkers detected. For example, a first sample can be obtained prior to the subject receiving treatment and one or more subsequent samples are taken after or during treatment of the subject. Changes in biomarker levels across the samples may provide an indication as to the effectiveness of the therapy.

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### Example 1: A Sensitive, High-throughput Cell-based Assay to Detect Mutant Huntingtin Seeding Activities in HD Patient CSF

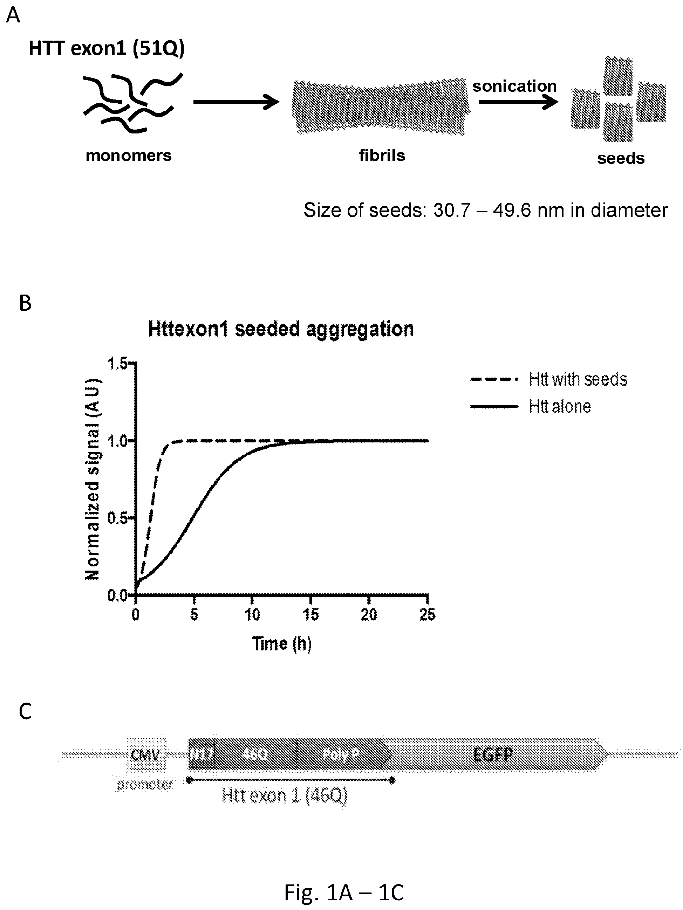

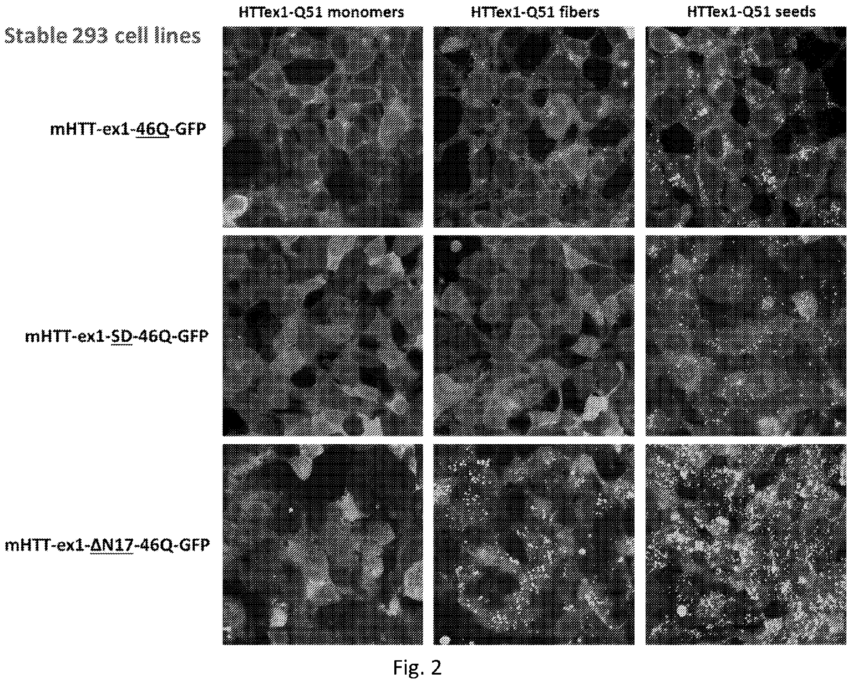

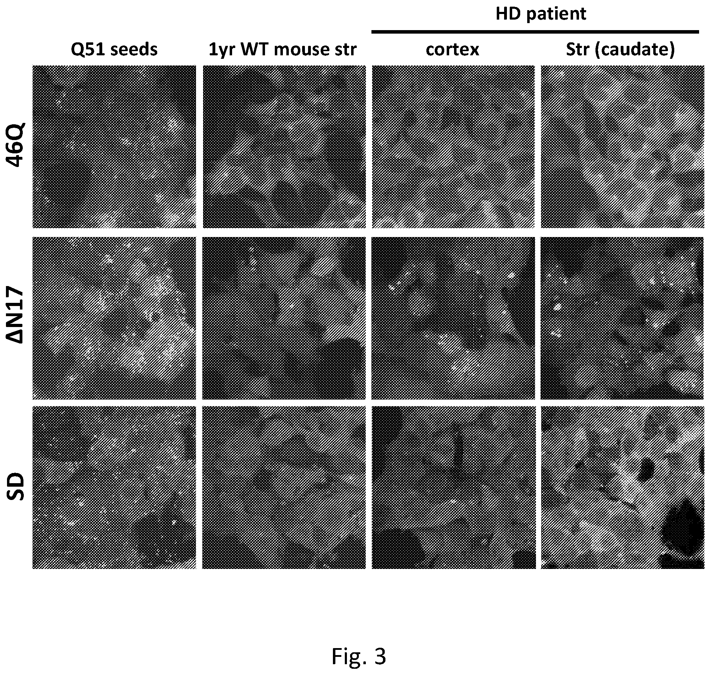

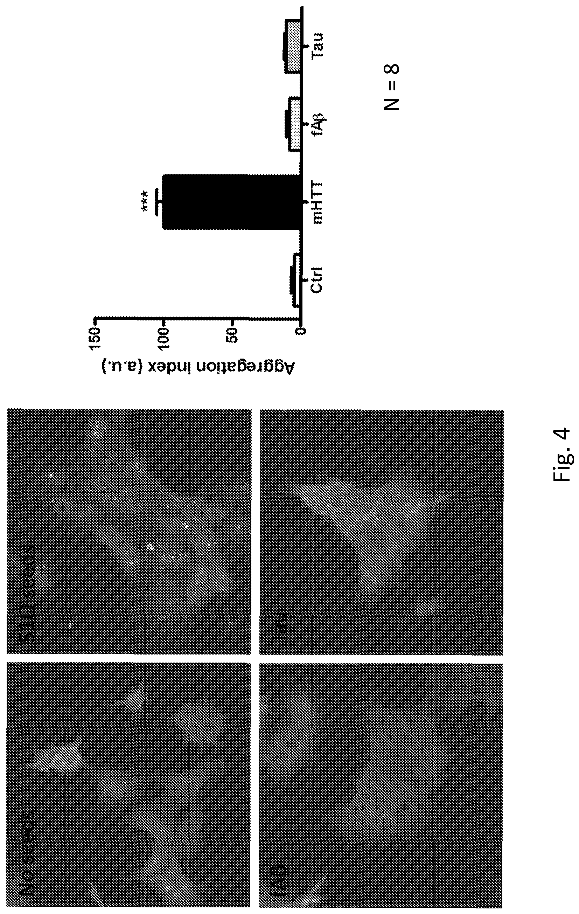

Described herein is the development of a cell-based HTT seeding assay. A DNA construct encoding Exon1 of mutant human HTT with a 46 polyQ repeat was designed (Figure 1C). This mutant HTT was engineered to delete amino acid residues 2-16 and was fused with a green fluorescent protein (GFP) at its C-terminus, named HTT-ΔN17-46Q-GFP hereafter. The HTT-ΔN17-46Q-GFP was then cloned into a pcDNA3 vector and transfected into HEK293 cells. Clones of stably transfected cells expressing HTT-ΔN17-46Q-GFP were selected to generate individual cell lines. Expression of the transgene in the cell lines was characterized to confirm its morphology, expression level and subcellular localization. Line 1E, named ΔN17-1E cells, was selected based on it moderate transgene expression with minimal protein aggregates at the baseline. Based on this cell line, a cell-based HTT seeding assay was developed. Extracellular application of preformed HTT aggregates or biosamples from HD patients as "seeds", to ΔN17-1E cells could induce intracellular aggregation of HTT-ΔN17-46Q-GFP proteins (Figure 1 through Figure 3). This seeding-induced aggregation event is similar to in vitro HTT aggregation, which preformed aggregates, or seeds, nucleates and facilitates the aggregation of HTT exon 1 monomers. Moreover, this activity is specific to utilizing aggregates of polyQ proteins since neither aggregates of β-amyloid and Tau nor biosamples from AD and PD patients could elicit HTT-ΔN17-46Q-GFP aggregation (Figure 4). The level of induced aggregates positively correlates with the amount of recombinant seeds added. Importantly, the seedability of the biosamples also correlates with the disease stage of the patients. Currently, the assay has been optimized to be performed in a high-throughput format, making it more capable with large quantity of samples. Thus, this cell-based seeding assay could be extremely useful for detecting seedible species in the biosamples, which is shown by us to increase as the disease progressing, and thus could be used as a biomarker for HD diagnosis and clinical trials, as well as for drug discovery.

The results of the experiments are now described.

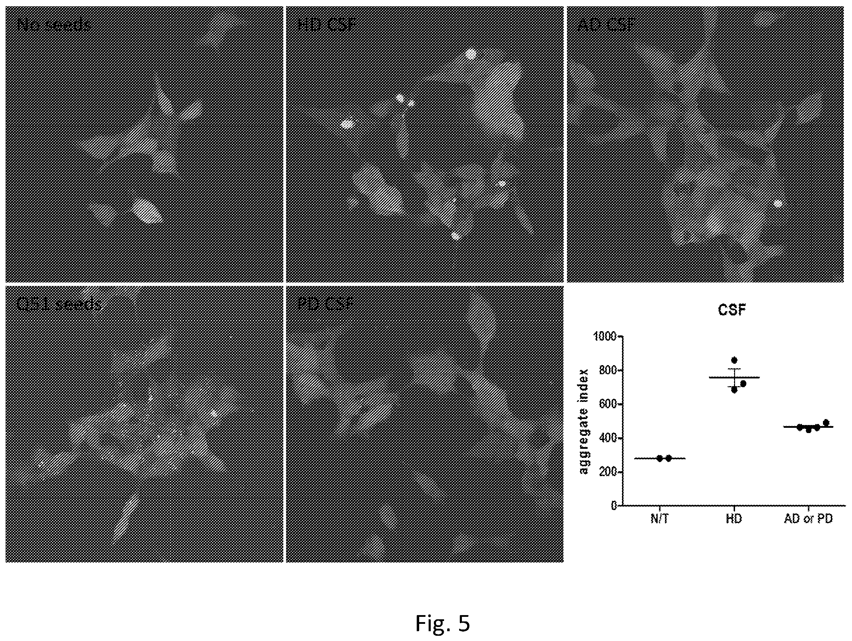

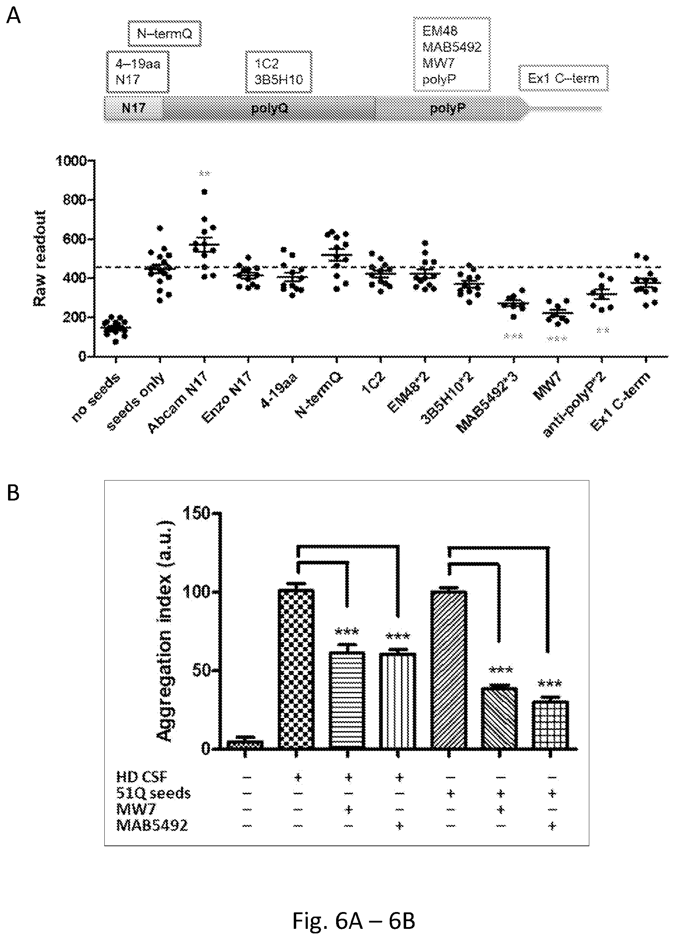

The cell-based assay forms very few, if any, aggregates by itself, but forms HTT-ΔN17-46Q-GFP aggregates upon the addition of preformed mHTT-exon1 seeds (derived from in vitro formed mHTT fibrils by sonication) or brain tissues from HD but not control postmorterm brain tissues (Figure 4). Importantly, the assay is specific to mHTT species (in vitro or using HD patient brain and CSF samples), since seeding with fibrillar Aβ, phosphorylated tau, or CSF from non-HD patients did not elicit the aggregation (Figure 4 and Figure 5). In addition, the HTT-ΔN17-46Q-GFP aggregation induced by preformed mHTT-exon1 seeds could be blocked by the antibodies specifically against the polyproline domain (polyP) in the mHTT exon 1, but not by the antibodies against the N17 or polyQ domains of mHTT (Figure 6A). Immuno-depletion of seedable mHTT species from the solution and CSF also significantly reduced the HTT-ΔN17-46Q-GFP aggregation (Figure 6B). The results further support the cell-based assay is specifically detecting seedable mHTT species in the biosamples, and can be used to characterize reagents or candidate therapeutic to deplete and/or block such species.

The cell- based seeding assay was performed using CSF samples from HD patients to measure the seedability of patient CSF on inducing HTT-ΔN17-46Q-GFP aggregation (Figure 7 through Figure 9). All 60 CSF samples were applied to cells in one experiment to reduce batch-to-batch difference. Five replicates per sample were performed to reduce procedure errors. As the CSF samples were provided in duplicate from 30 samples, the replicability of the duplicates was first evaluated. As shown in Figure 7A, the duplicates induced similar reaction of HTT-ΔN17-46Q-GFP aggregation. The correlation of the seedability (aggregate index) of CSF with DCL scores, CAP score and CAG length was then evaluated. There is no CAG length-dependency for seedability (Figure 7B). However, seedability of CSF significantly increased when the patient is diagnosed or near-onset (DCL = 4) of HD (Figure 7C and Figure 7D).

An important comparison is to compare the seedability of CSF from two visits for the same individuals. When they were grouped by the DCL score of the first visit, Figure 8 clearly shows that there is increase of seedability as disease progress, when the individual's DCL score of first visit is higher than 1. And the difference also increases as the DCL score gets higher. This demonstrates that there is a correlation of seedable mHTT species in CSF with the disease progression. It also raises the possibility of using CSF mHTT as a biomarker for disease status, as the cell-based aggregation assay could sensitively detect CSF mHTT.

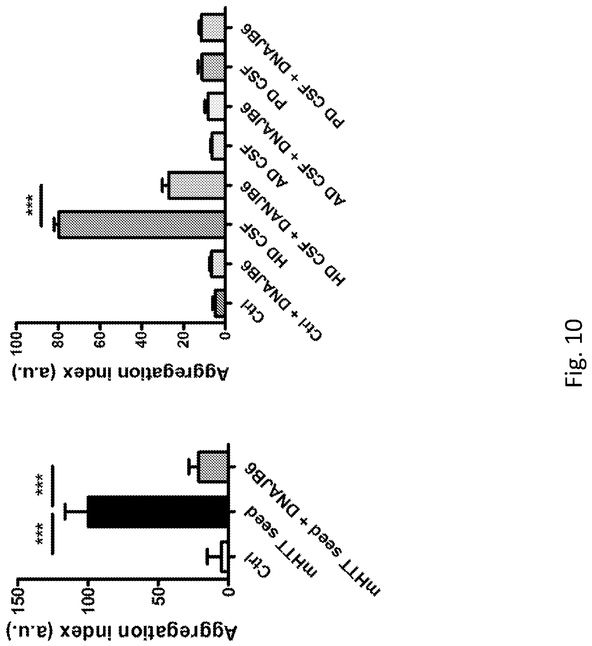

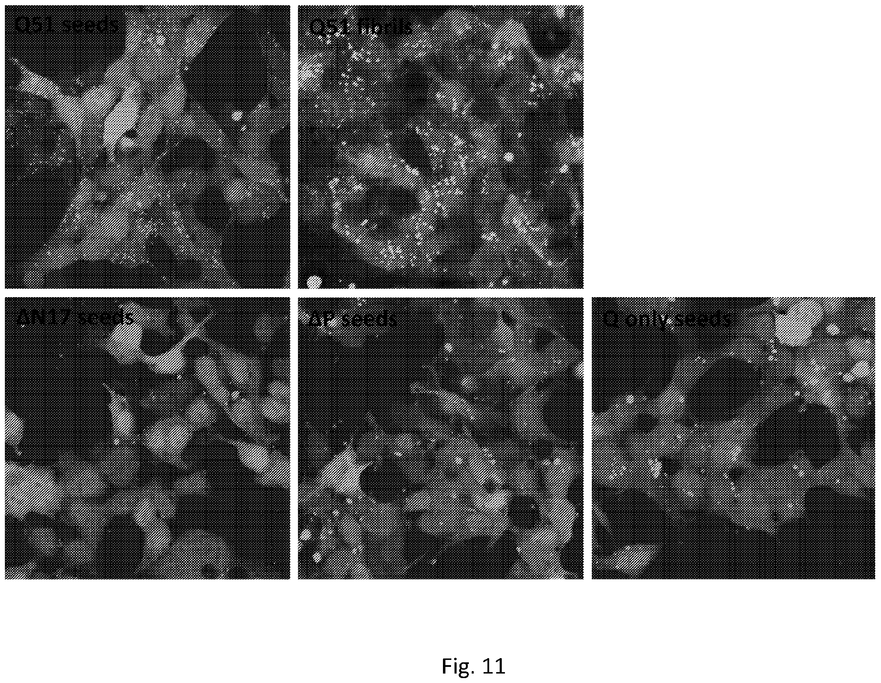

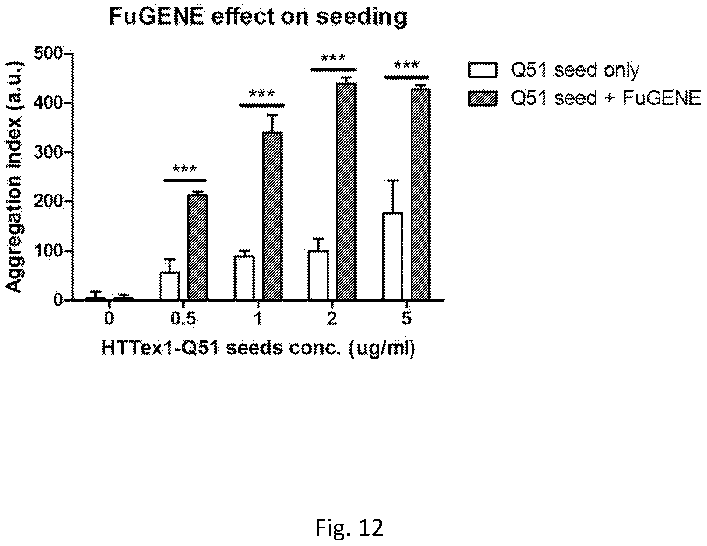

Moreover, DNAJB6 of the Hsp40 family has been shown to efficiently suppress polyQ aggregation through inhibition of the early step of amyloid fibril formation (Kakkar V et al (2016) Mol Cell 62: 272-283). Overexpression of DNAJB6 in ΔN17 cells by transit transfection dramatically reduced HTT-ΔN17-46Q-GFP aggregation induced by preformed mHTT-exon1 seeds and HD CSF (Figure 10). Also, the morphology of HTT-ΔN17-46Q-GFP aggregates is related to the conformation of the seeds administered (Figure 11). Finally, the possibility of further enhancing the sensitivity of the assay, e.g. by addition FuGENE (a transfection reagent from Promega) is demonstrated (Figure 12). These results support the potential use of the cell-based assay for screenings for small molecules and genes that could modulate (e.g. blocking) mHTT aggregation, thus has high potential to serve as an assay for high-throughput therapeutic screening against mHTT seeding activities elicited by either the in vitro formed seeds or patient biosamples.

### Sequences:

SEQ ID NO:1: HTTex1-46Q-EGFP nucleic acid sequence
SEQ ID NO:2: HTTex1-46Q-EGFP amino acid sequence
SEQ ID NO:3: HTTex1-46Q-EGFP nucleic acid sequence lacking encoding sequence for residues 2-16 of SEQ ID NO:2 (N17)
SEQ ID NO:4: HTTex1-46Q-EGFP amino acid sequence lacking sequence for residues 2-16 of SEQ ID NO:2 (N17)
SEQ ID NO:5: nucleic acid sequence for a fragment of exon 1 of HTT encoding a 46 polyQ region and a proline rich region
SEQ ID NO:6: amino acid sequence for a fragment of exon 1 of HTT comprising a 46 polyQ region and a proline rich region

## Claims

1. A nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

2. A cell comprising a nucleic acid molecule comprising a nucleotide sequence encoding a protein comprising a mutant huntingtin protein (mHTT) or fragment thereof, wherein the mHTT comprises a fragment of exon 1 of HTT comprising a polyQ region consisting of 46 consecutive glutamine amino acids.

3. The cell of claim 2, wherein the cell is a mammalian cell, or is an isolated cell.

4. The cell of claim 2, wherein the protein is a fusion protein comprising a detectable protein, optionally wherein the detectable protein is at least one protein selected from the group consisting of a fluorescent protein, an enzyme, and an epitope, further optionally wherein the fluorescent protein is selected from the group consisting of a red fluorescent protein, a green fluorescent protein, a blue fluorescent protein, a yellow fluorescent protein, and a cyan fluorescent protein, further optionally wherein the fluorescent protein is EGFP.

5. An isolated protein comprising a mutant huntingtin protein (mHTT) or fragment thereof, wherein the mHTT comprises a fragment of exon 1 of HTT comprising a polyQ region consisting of 46 consecutive glutamine amino acids.

6. The nucleic acid molecule of claim 1, the cell of claim 2, or the isolated protein of claim 5, wherein the mHTT comprises a comprises a sequence selected from the group consisting of SEQ ID NO:2, SEQ ID NO:4 and SEQ ID NO:6.

7. The nucleic acid molecule of claim 1, the cell of claim 2, or the isolated protein of claim 5, wherein the mHTT is encoded by a sequence selected from the group consisting of SEQ ID NO:1, SEQ ID NO:3 and SEQ ID NO:5.

8. The nucleic acid molecule of claim 1, the cell of claim 2, or the isolated protein of claim 5, wherein the mHTT lacks amino acid residues 2 through 16 of exon 1 of HTT.

9. The nucleic acid molecule of claim 1, wherein the nucleotide sequence encoding the mHTT is operably linked to a nucleotide sequence encoding a detectable marker, optionally wherein the detectable marker is at least one marker selected from the group consisting of a fluorescent protein, an enzyme, and an epitope.

10. The isolated protein of claim 5, wherein the protein is a fusion protein comprising an amino acid sequence of mHTT fused to an amino acid sequence of a detectable marker, optionally wherein the detectable marker is at least one marker selected from the group consisting of a fluorescent protein, an enzyme, and an epitope.

11. A method of identifying a compound that prevents or treats at least one of huntingtin aggregation, seeding and cytoxicity, the method comprising: culturing the cell of claim 2 in the presence of a candidate agent under conditions that allow for aggregation of the mHTT, measuring aggregation of the mHTT in the presence of the candidate agent, and comparing mHTT aggregation measured in the presence of the candidate agent to mHTT aggregation measured in the absence of the candidate agent, wherein if mHTT aggregation measured is reduced in the presence of the candidate agent as compared to in the absence of the candidate agent, then the candidate agent is identified as a compound that prevents or treats mHTT aggregation.

12. The method of claim 11, wherein the conditions that allow for aggregation of the mHTT comprise contacting the cell with a sample from a subject having HD.

13. A method of diagnosing a subject as having Huntington's Disease (HD) comprising contacting a cell of claim 2 with a sample from a subject, detecting aggregation of the mHTT in the presence of the sample, and diagnosing the subject as having HD or at risk of developing HD when mHTT aggregation is detected.

14. The method of claim 13, wherein the sample is selected from the group consisting of CSF and blood.

## Patentansprüche

1. Nukleinsäuremolekül, umfassend eine Nukleotidsequenz, die ein ein mutiertes Huntingtin-Protein (mHTT) oder Fragment davon umfassendes Protein codiert, wobei das mHTT ein Fragment aus Exon 1 des HTT umfasst, das eine polyQ-Region umfasst, die aus 46 aufeinanderfolgenden Glutaminaminosäuren besteht.

2. Zelle, umfassend ein Nukleinsäuremolekül, das eine Nukleotidsequenz umfasst, die ein ein mutiertes Huntingtin-Protein (mHTT) oder Fragment davon umfassendes Protein codiert, wobei das mHTT ein Fragment aus Exon 1 des HTT umfasst, das eine polyQ-Region umfasst, die aus 46 aufeinanderfolgenden Glutaminaminosäuren besteht.

3. Zelle nach Anspruch 2, wobei es sich bei der Zelle um eine Säugetierzelle oder eine isolierte Zelle handelt.

4. Zelle nach Anspruch 2, wobei es sich bei dem Protein um ein Fusionsprotein handelt, das ein nachweisbares Protein umfasst, gegebenenfalls wobei es sich bei dem nachweisbaren Protein um mindestens ein Protein handelt, das aus der Gruppe bestehend aus einem Fluoreszenzprotein, einem Enzym und einem Epitop ausgewählt ist, ferner gegebenenfalls wobei das Fluoreszenzprotein aus der Gruppe bestehend aus einem roten Fluoreszenzprotein, einem grünen Fluoreszenzprotein, einem blauen Fluoreszenzprotein, einem gelben Fluoreszenzprotein und einem cyanblauen Fluoreszenzprotein ausgewählt ist, ferner gegebenenfalls wobei es sich bei dem Fluoreszenzprotein um EGFP handelt.

5. Isoliertes Protein, umfassend ein mutiertes Huntingtin-Protein (mHTT) oder Fragment davon, wobei das mHTT ein Fragment aus Exon 1 des HTT umfasst, das eine polyQ-Region umfasst, die aus 46 aufeinanderfolgenden Glutaminaminosäuren besteht.

6. Nukleinsäuremolekül nach Anspruch 1, Zelle nach Anspruch 2 oder isoliertes Protein nach Anspruch 5, wobei das mHTT eine Sequenz umfasst, die aus der Gruppe bestehend aus SEQ ID NO:2, SEQ ID NO:4 und SEQ ID NO:6 ausgewählt ist.

7. Nukleinsäuremolekül nach Anspruch 1, Zelle nach Anspruch 2 oder isoliertes Protein nach Anspruch 5, wobei das mHTT von einer Sequenz codiert wird, die aus der Gruppe bestehend aus SEQ ID NO:1, SEQ ID NO:3 und SEQ ID NO:5 ausgewählt ist.

8. Nukleinsäuremolekül nach Anspruch 1, Zelle nach Anspruch 2 oder isoliertes Protein nach Anspruch 5, wobei dem mHTT Aminosäurereste 2 bis 16 aus Exon 1 des HTT fehlen.

9. Nukleinsäuremolekül nach Anspruch 1, wobei die das mHTT codierende Nukleotidsequenz funktionell mit einer einen nachweisbaren Marker codierenden Nukleotidsequenz verknüpft ist, gegebenenfalls wobei es sich bei dem nachweisbaren Marker um mindestens einen Marker handelt, der aus der Gruppe bestehend aus einem Fluoreszenzprotein, einem Enzym und einem Epitop ausgewählt ist.

10. Isoliertes Protein nach Anspruch 5, wobei es sich bei dem Protein um ein Fusionsprotein handelt, das eine mHTT-Aminosäuresequenz fusioniert an eine Aminosäuresequenz eines nachweisbaren Markers umfasst, gegebenenfalls wobei es sich bei dem nachweisbaren Marker um mindestens einen Marker handelt, der aus der Gruppe bestehend aus einem Fluoreszenzprotein, einem Enzym und einem Epitop ausgewählt ist.

11. Verfahren zum Identifizieren einer Verbindung, die mindestens einer aus Huntingtin-Aggregation, -"Seeding" und -Zytotoxizität vorbeugt oder diese behandelt, wobei das Verfahren Folgendes umfasst: Kultivieren der Zelle nach Anspruch 2 in Gegenwart eines Kandidatenagens unter Bedingungen, die Aggregation des mHTT ermöglichen, Messen von Aggregation des mHTT in Gegenwart des Kandidatenagens und Vergleichen von in Gegenwart des Kandidatenagens gemessener mHTT-Aggregation mit in Abwesenheit des Kandidatenagens gemessener mHTT-Aggregation, wobei das Kandidatenagens als Verbindung, die mHTT-Aggregation vorbeugt oder behandelt, identifiziert wird, wenn die gemessene mHTT-Aggregation in Gegenwart des Kandidatenagens verglichen mit mHTT-Aggregation in Abwesenheit des Kandidatenagens reduziert ist.

12. Verfahren nach Anspruch 11, wobei die Bedingungen, die Aggregation des mHTT ermöglichen, Inkontaktbringen der Zelle mit einer Probe aus einem Individuum mit HD umfassen.

13. Verfahren zum Diagnostizieren eines Individuums mit Chorea Huntington (HD= Huntington's Disease), umfassend Inkontaktbringen einer Zelle nach Anspruch 2 mit einer Probe aus einem Individuum, Nachweisen von Aggregation des mHTT in Gegenwart der Probe und Diagnostizieren des Individuums mit HD oder als risikobehaftet, HD zu entwickeln, sofern mHTT-Aggregation nachgewiesen wurde.

14. Verfahren nach Anspruch 13, wobei die Probe aus der Gruppe bestehend aus CSF und Blut ausgewählt ist.

## Revendications

1. Molécule d'acide nucléique comprenant une séquence nucléotidique codant pour une protéine comprenant une protéine huntingtine mutante (mHTT) ou un fragment de celle-ci, dans laquelle la mHTT comprend un fragment de l'exon 1 de HTT comprenant une région polyQ constituée de 46 acides aminés glutamine consécutifs.

2. Cellule comprenant une molécule d'acide nucléique comprenant une séquence nucléotidique codant pour une protéine comprenant une protéine huntingtine mutante (mHTT) ou un fragment de celle-ci, dans laquelle la mHTT comprend un fragment de l'exon 1 de HTT comprenant une région polyQ constituée de 46 acides aminés glutamine consécutifs.

3. Cellule selon la revendication 2, dans laquelle la cellule est une cellule de mammifère, ou est une cellule isolée.

4. Cellule selon la revendication 2, dans laquelle la protéine est une protéine de fusion comprenant une protéine détectable, éventuellement dans laquelle la protéine détectable est au moins une protéine choisie dans le groupe constitué par une protéine fluorescente, une enzyme et un épitope, en outre éventuellement dans laquelle la protéine fluorescente est choisie dans le groupe constitué par une protéine fluorescente rouge, une protéine fluorescente verte, une protéine fluorescente bleue, une protéine fluorescente jaune, et une protéine fluorescente cyan, en outre éventuellement dans laquelle la protéine fluorescente est EGFP.

5. Protéine isolée comprenant une protéine huntingtine mutante (mHTT) ou un fragment de celle-ci, dans laquelle la mHTT comprend un fragment de l'exon 1 de HTT comprenant une région polyQ constituée de 46 acides aminés glutamine consécutifs.

6. Molécule d'acide nucléique selon la revendication 1, cellule selon la revendication 2, ou protéine isolée selon la revendication 5, dans laquelle la mHTT comprend une séquence choisie dans le groupe constitué par SEQ ID NO:2, SEQ ID NO:4 et SEQ ID NO:6.

7. Molécule d'acide nucléique selon la revendication 1, cellule selon la revendication 2, ou protéine isolée selon la revendication 5, dans laquelle la mHTT est codée par une séquence choisie dans le groupe constitué par SEQ ID NO:1, SEQ ID NO:3 et SEQ ID NO:5.

8. Molécule d'acide nucléique selon la revendication 1, cellule selon la revendication 2, ou protéine isolée selon la revendication 5, dans laquelle la mHTT est dépourvue des résidus d'acides aminés 2 à 16 de l'exon 1 de la HTT.

9. Molécule d'acide nucléique selon la revendication 1, dans laquelle la séquence nucléotidique codant pour la mHTT est liée de manière fonctionnelle à une séquence nucléotidique codant pour un marqueur détectable, éventuellement dans laquelle le marqueur détectable est au moins un marqueur choisi dans le groupe constitué par une protéine fluorescente, une enzyme et un épitope.

10. Protéine isolée selon la revendication 5, dans laquelle la protéine est une protéine de fusion comprenant une séquence d'acides aminés de mHTT fusionnée à une séquence d'acides aminés d'un marqueur détectable, éventuellement dans laquelle le marqueur détectable est au moins un marqueur choisi dans le groupe constitué par une protéine fluorescente, une enzyme et un épitope.

11. Procédé d'identification d'un composé qui prévient ou traite au moins l'un parmi l'agrégation de la huntingtine, l'ensemencement et la cytotoxicité, le procédé comprenant : la culture de la cellule selon la revendication 2 en présence d'un agent candidat dans des conditions qui permettent l'agrégation de la mHTT, la mesure de l'agrégation de la mHTT en présence de l'agent candidat, et la comparaison de l'agrégation de la mHTT mesurée en présence de l'agent candidat à l'agrégation de la mHTT mesurée en l'absence de l'agent candidat, dans lequel si l'agrégation de la mHTT mesurée est réduite en présence de l'agent candidat par rapport à en l'absence de l'agent candidat, alors l'agent candidat est identifié comme un composé qui prévient ou traite l'agrégation de la mHTT.

12. Procédé selon la revendication 11, dans lequel les conditions qui permettent l'agrégation de la mHTT comprennent la mise en contact de la cellule avec un échantillon provenant d'un sujet ayant HD.

13. Procédé de diagnostic d'un sujet comme ayant la maladie de Huntington (HD) comprenant la mise en contact d'une cellule selon la revendication 2 avec un échantillon provenant d'un sujet, la détection de l'agrégation de la mHTT en présence de l'échantillon, et le diagnostic du sujet comme ayant une HD ou risquant de développer une HD lorsqu'une agrégation de mHTT est détectée.

14. Procédé selon la revendication 13, dans lequel l'échantillon est choisi dans le groupe constitué par CSF et le sang.
